(19) Europäisches Patentamt / European Patent Office / Office européen des brevets

(11) **EP 2 457 510 A1**

(12) **EUROPÄISCHE PATENTANMELDUNG**

(43) Veröffentlichungstag:
**30.05.2012 Patentblatt 2012/22**

(51) Int Cl.:
***A61B 5/145*** *(2006.01)* ***A61B 5/05*** *(2006.01)*

(21) Anmeldenummer: **11190552.7**

(22) Anmeldetag: **24.11.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **24.11.2010 EP 10192469**
**24.11.2010 EP 10192470**
**24.11.2010 EP 10192472**
**24.11.2010 EP 10192473**

(71) Anmelder:
• **eesy-id GmbH**
**82166 Gräfelfing (DE)**

• **Friedrich-Alexander-Universität Erlangen-Nürnberg**
**91054 Erlangen (DE)**

(72) Erfinder:
• **Fischer, Georg**
**90425 Nürnberg (DE)**
• **Hofmann, Maximilian**
**90419 Nürnberg (DE)**

(74) Vertreter: **Klinski, Robert**
**PATENTSHIP**
**Patentanwaltskanzlei**
**Elsenheimerstraße 65**
**80687 München (DE)**

(54) **Erfassungsvorrichtung zum Erfassen eines Blutbildparameters**

(57) Die Erfindung betrifft eine Erfassungsvorrichtung (100) zum Erfassen eines Blutbildparameters von Blut in einem Blutgefäß (102), mit einer elektrischen Leitungsanordnung (104), welche an das Blutgefäß (102) dielektrisch koppelbar ist, einem Netzwerkanalysator (106) zum Anregen der elektrischen Leitungsanordnung (104), so dass ein elektro-magnetisches Feld (108) von der elektrischen Leitungsanordnung (104) ausgeht, und zum Erfassen einer Änderung des elektro-magnetischen Feldes (108) bei dielektrischer Belastung der elektrischen Leitungsanordnung (104) mit dem Blutgefäß (102) und zum Erfassen des Blutbildparameters auf der Basis der erfassten Änderung des elektromagnetischen Feldes (108).

Fig. 1

EP 2 457 510 A1

**Beschreibung**

Die vorliegende Erfindung betrifft das Gebiet der Erfassung einer Konzentration eines Blutbestandteiles, beispielsweise einer Konzentration von Blutzucker.

Zur Bestimmung eines Blutbildparameters wie beispielsweise einer Konzentration eines Blutbestandteils kann invasiv Blut entnommen werden. Der Blutbildparameter kann dann unter Verwendung des entnommenen Bluts anhand standardisierter Teststreifen bestimmt werden, deren elektrische Widerstandswerte von der Konzentration des Blutbestandteiles, beispielsweise Blutzuckers, abhängen. Der jeweilige elektrische Widerstandswert kann beispielsweise unter Verwendung eines Blutzuckermessgerätes, das eine Gleichstrom-Widerstandsmessung zur Erfassung eines elektrischen Widerstandswertes eines Testreifens durchführt, erfasst werden. Der Widerstandswert kann anhand eines an sich bekannten Zusammenhanges zwischen einer Blutzuckerkonzentration und einem Widerstandswert in eine Blutzuckerkonzentration umgerechnet werden. Zur Erzielung einer hohen Erfassungsgenauigkeit ist jeder Teststreifen mit Kalibrierdaten, beispielsweise mit einem Referenzwiderstandswert oder mit einer entsprechenden Codierung, versehen, wodurch Variationen von Eigenschaften der Teststreifen ausgeglichen werden können. Nachteilig an invasiven Verfahren ist jedoch die Notwendigkeit der Blutentnahme und somit einer Verletzung eines Patienten. Außerdem ist eine kontinuierliche Erfassung einer Konzentration eines Blutbestandteils, um beispielsweise dessen Tagesgangkurve zu ermitteln, aufwändig. Darüber hinaus kann mittels des invasiven Verfahrens eine Zeitverzögerung zwischen einer Nahrungsaufnahme und beispielsweise einem Anstieg des Blutzuckers nicht genau erfasst werden. Ferner kann insbesondere bei einer geringen Konzentration des Blutzuckers im Blut ein Zeitpunkt der Insulinabgabe an den Patienten nicht genau bestimmt werden.

Zur nichtinvasiven Bestimmung eines Blutbildparameters wie beispielsweise einer Stoffkonzentration oder einer Stoffzusammensetzung im Blut können mikrowellenspektroskopische Verfahren eingesetzt werden. Die Mikrowellenspektroskopie zur Erfassung von Blutbildparametern basiert auf einer Einkopplung eines Mikrowellensignals in ein blutdurchströmtes Gewebe und einer Erfassung einer frequenzabhängigen Absorption eingekoppelter Mikrowellenenergie. In den Druckschriften von Buford Randal Jean et al., "A microwave frequency sensor for non-invasive blood-glucose measurement", SAS 2008 - IEEE Sensors Applications Symposium, Atlanta, GA, February 12 - 14, 2008, and M. McClung, Calibration methodology for a microwave non-invasive glucose sensor, Master Thesis, Baylor University, Mai 2008, ist eine Elektrodenanordnung zur Bestimmung einer Blutzuckerkonzentration beschrieben. Dabei wird ausgenutzt, dass die dielektrischen Eigenschaften von Blut von einem Blutzuckergehalt abhängen. Durch Anpressen eines Daumens auf den Mikrowellensensor wird eine Änderung der Dielektrizitätszahl des Daumens durch eine Verstimmung eines Resonators gemessen. Durch das Anpressen des Daumes wird jedoch Blut verdrängt, was zu einer Verfälschung der Messergebnisse führen kann. Des Weiteren können die Messungen nicht kontinuierlich durchgeführt werden. Die Auswertung der Messdaten zur Bestimmung des Blutzuckergehalts hängt zudem von dem jeweiligen Patienten ab und ist daher bei anderen Patienten nicht reproduzierbar. Darüber hinaus ist mit diesem Verfahren die Eindringtiefe der Mikrowellenleistung nicht steuerbar, so dass eine Unterscheidung zwischen kapillarem und venösem Blut nicht möglich ist. Ferner wird die Änderung der Dielektrizitätszahl auf der Basis einer Eintormessung durchgeführt, welche anfällig bezüglich Fehlanpassungen ist.

Es ist die Aufgabe der vorliegenden Erfindung, ein effizientes Konzept zur mikrowellenbasierten, nicht-invasiven Bestimmung eines Blutbildparameters, insbesondere einer Konzentration von Blutzucker, in einem durch ein Blutgefäß fließendes Blut zu schaffen.

Diese Aufgabe wird durch die Merkmale der unabhängigen Ansprüche gelöst. Vorteilhafte Weiterbildungsformen sind Gegenstand der abhängigen Ansprüche.

Die Erfindung basiert auf der Erkenntnis, dass eine dielektrische Belastung einer angeregten elektrischen Leitungsanordnung, beispielsweise einer koplanaren Mikrostreifenleitung, mit einem Blutgefäß zu einer Änderung des elektro-magnetischen Feldes der Leitungsanordnung führt. Eine Erfassung dieser Änderung kann für die Bestimmung eines Blutbildparameters ausgenutzt werden. Ist der zu bestimmende Blutbildparameter beispielsweise eine Konzentration von Blutzucker im Blut, so ist eine Änderung des elektromagnetischen Feldes der Leitungsanordnung ein Maß für die Konzentration des Blutzuckers, d.h. für den Blutzuckerspiegel. Die Erfassung des Blutbildparameters auf der Basis der Änderung des elektro-magnetischen Feldes basiert auf der weiteren Erkenntnis, dass die Viskosität einer Wasserlösung mit zunehmender Zuckerkonzentration sich ändern kann, und sich damit auf die Impedanz der Leitungsanordnung auswirkt. Auf diese Weise kann durch eine Erfassung einer Änderung des elektro-magnetischen Feldes, beispielsweise durch eine Streuparametermessung in einem Frequenzbereich von beispielsweise 1 bis 100 GHz die Konzentration des Blutzuckers erfasst werden.

Die Erfindung basiert auf der weiteren Erkenntnis, dass ein Blutgefäß wie beispielsweise eine Vene oder eine Arterie, das dieses Blutgefäß umgebende Fettgewebe sowie die darüber liegende Hautschicht als ein dielektrisches Wellenleitersystem aufgefasst werden können. Bei einer Anregung eines derartigen dielektrischen Wellenleitersystems mit einer Leitungsanordnung, wie beispielsweise einer Mikrostreifenleitung oder einem Hohlleiter, können daher unterschiedliche Moden bzw. Wellentypen angeregt werden, beispielsweise eine transversal-elektromagnetische (TEM) Welle oder transversal-elektrische (TE) Welle oder transversal-magnetische (TM) Welle oder eine HE-Welle. Bei einer TE-Welle existiert eine in Ausbreitungsrichtung weisende, von Null verschiedene Komponente des Magnetfeldes. Bei einer TM-Welle existiert hingegen eine in Modenausbreitungsrichtung weisende, von Null verschiedene Komponente eines elektrischen Feldes. In Abhängigkeit von einer Hochfrequenzanregung durch die Leitungsanordnung können daher in einem das Blutgefäß und die Hautschicht umfassenden dielektrischen Wellenleitersystem unterschiedliche, auch in Blutflussrichtung ausbreitungsfähige Moden angeregt werden, die zu einer Belastung der Leitungsanordnung führen, wodurch eine genaue Erfassung eines Blutbildparameters möglich ist.

Gemäß einem Aspekt betrifft die Erfindung eine Erfassungsvorrichtung zum Erfassen eines Blutbildparameters von Blut in einem Blutgefäß mit einer elektrischen Leitungsanordnung, welche an das Blutgefäß dielektrisch koppelbar ist, einem Netzwerkanalysator zum Anregen der elektrischen Leitungsanordnung, so dass ein elektro-magnetisches Feld von der elektrischen Leitungsanordnung ausgeht. Der Netzwerkanalysator ist ferner zum Erfassen einer Änderung des elektro-magnetischen Feldes bei dielektrischer Belastung der elektrischen Leitungsanordnung mit dem Blutgefäß und zum Erfassen des Blutbildparameters auf der Basis der erfassten Änderung des elektro-magnetischen Feldes vorgesehen.

Die Änderung des elektro-magnetischen Feldes kann beispielsweise anhand einer Referenzgröße ermittelt werden, beispielsweise einer Referenz-Impedanz eines als Referenz verwendeten elektro-magnetischen Feldes. Referenzmessungen können beispielsweise mit Wasser gemacht werden, um die Ausbreitungscharakteristik des elektro-magnetischen Feldes in Wasser zu bestimmen.

So kann die Änderung des elektro-magnetischen Feldes bei Belastung mit einem Wassergefäß als Referenzmessung bestimmt werden. Das Wasser in dem Wassergefäß kann dann nach und nach beispielsweise mit Zucker oder mit einem anderen die Absorption des elektro-magnetischen Feldes beeinflussenden Stoff versetzt werden, um weitere Referenzmessungen zur Ermittlung von Referenz-Impedanzen zu erhalten. Zusätzlich oder alternativ können Referenzmessungen am realen Blutgefäß vorgenommen werden, bei denen bekannt ist, wie der Verlauf des Blutbildparameters aussieht. Durch Vergleich der so erhaltenen Referenzmessungen am realen Blutgefäß oder/und der Referenzmessungen an dem mit Zucker bzw. dem oben genannten Stoff versetzten Wassergefäß kann einer Zuordnung der gemessenen Impedanzen zu dem Blutbildparameter vorgenommen werden.

Gemäß einer Ausführungsform betrifft die Erfindung eine Erfassungsvorrichtung, Erfassungsvorrichtung, wobei bei dielektrischer Kopplung der elektrischen Leitungsanordnung an das Blutgefäß ein überwiegender Energiefluss des elektromagnetischen Feldes in dem Blutgefäß konzentriert ist. Der Energiefluss ist beispielsweise dann überwiegend in dem Blutgefäß konzentriert, wenn mehr als 50%, beispielsweise 60%, 70%, 80% oder 90%, des Energieflusses in dem Blutgefäß konzentriert ist.

Das elektro-magnetische Feld der Leitungsanordnung dringt in die Ader ein und ist dort zumindest teilweise konzentriert. Damit ist sichergestellt, dass die Messstrecke nicht aus einem eher zufälligen Gemisch verschiedener Stoffe, beispielsweise Fett, Knochen, Haut und Blut besteht, sondern im Wesentlichen nur aus Blut, wie bereits ausführlich in den Anmeldungen EP 10192470 und EP 10192473 beschrieben ist, deren Offenbarungsgehalt dieser Beschreibung insofern zuzurechnen ist.

Gemäß einer Ausführungsform betrifft die Erfindung eine Erfassungsvorrichtung, wobei die dielektrische Kopplung der elektrischen Leitungsanordnung an das Blutgefäß durch nichtinvasives Befestigen der elektrischen Leitungsanordnung an einer Hautoberfläche eines menschlichen oder tierischen Körpers erfolgt.

Das Befestigen an der Hautoberfläche kann ein reines Auflegen auf die Haut sein, beispielsweise bei Verwendung eines dünnen Mikrostreifenleiters, der sich an die Unebenheiten der Hautoberfläche anschmiegt. Vorzugsweise wird zum Befestigen aber eine Befestigungseinrichtung verwendet, um für einen geeigneten Anpressdruck zu sorgen, damit die Messungen die erforderliche Genauigkeit aufweisen. Beispielsweise kann eine Anpressmanschette verwendet werden, wie sie bei Blutdruckmessgeräten verwendet wird. Gemäß einer Ausgestaltung der Ausführungsform umfasst die Erfassungsvorrichtung ein an einem Arm befestigbares Armband gemäß der Druckschrift EP 10192472, deren diesbezüglicher Offenbarungsgehalt dieser Offenbarung zuzurechnen ist, mit der Erfassungsvorrichtung gemäß den in dieser Anmeldung beschriebenen Ausführungsformen und mit einer Einstellvorrichtung zum Einstellen eines vorgegebenen Anpressdruckes des Armbandes auf den Arm.

Gemäß einer Ausführungsform betrifft die Erfindung eine Erfassungsvorrichtung, bei der der Blutbildparameter eine dielektrische und/oder magnetische ($\mu_r$) Leitfähigkeit des Blutes in dem Blutgefäß umfasst.

Die dielektrische Leitfähigkeit, auch Permittivität oder $\varepsilon_r$ genannt, ist ein charakteristischer Parameter für die Durchlässigkeit eines Materials für elektrische Felder und kann daher verwendet werden, um den Blutbildparameter zu charakterisieren. Beispielsweise ändert sich die Permittivität von Blut abhängig von der Zuckerkonzentration, so dass von der Permittivität auf die Zuckerkonzentration geschlossen werden kann, beispielsweise durch Vergleich mit Referenzmessungen, wie bereits oben erläutert.

Gemäß einer Ausführungsform betrifft die Erfindung eine Erfassungsvorrichtung, bei der der Blutbildparameter eine Zuckerkonzentration bzw. Glukosekonzentration des Blutes in dem Blutgefäß umfasst.

Gemäß einer Ausführungsform betrifft die Erfindung eine Erfassungsvorrichtung, bei der der Netzwerkanalysator ausgelegt ist, den Blutbildparameter basierend auf einer S-Parametermessung bzw. Streuparametermessung zu bestimmen.

Das liegende Blutgefäß kann als ein dielektrischer Wellenleiter interpretiert werden, in dem je nach Frequenz verschiedene Moden bzw. Wellentypen, beispielsweise eine TE-Welle, eine TM-Welle, eine TEM-Welle oder eine HE-Welle, ausbreitungsfähig sein können. Der Netzwerkanalysator kann derart ausgestaltet sein, dass er die elektrische Leitungsanordnung anregt, um dezidiert Mikrowellenleistung in das Blutgefäß einzukoppeln und diese beispielsweise nach einigen Zentimetern wieder auszukoppeln. Die Leitungsanordnung kann zu diesem Zweck als Mikrostreifenleitung ausgestaltet sein, so dass ein Leitungsstreifen als Erreger zur Einkopplung von Mikrowellenenergie dient und der andere Leitungsstreifen als Empfänger zum Empfangen der Mikrowellenenergie dient. Das Blutgefäß dient dabei als eine Messstrecke, das gleichzeitig jedoch eine Last für die Mikrowellenenergie darstellt und die Ausbreitung der elektro-magnetischen Feldlinien beeinflusst bzw. beeinträchtigt. Das Blutgefäß ist als ein verteiltes Element und nicht mehr als ein konzentriertes Element anzusehen. Bevorzugt wird die Messung der Feldänderung auf der Basis einer Zweitormessung durchgeführt. Dabei können Primärmoden in dem dielektrischen Wellenleiter, d.h. dem Blutgefäß, angeregt werden, so dass mit einer derartigen Messmethode die Erfassung des Blutbildparameters sehr präzise vorgenommen werden kann. Um Primärmoden im dielektrischen Wellenleitersystem anzuregen, kann berücksichtigt werden, dass je nach gewählter Frequenz eines Sendesignals unterschiedliche Moden dominant sein können. Bevorzugt sind Modentypen, die eine Konzentration der Felder in dem Blutgefäß aufweisen, solchen Moden vorzuziehen, bei denen die Felder in einer Hautschicht konzentriert sind. Aufgrund der dielektrischen Eigenschaften des Blutgefäßes zeigt sich, dass für bestimmte Modentypen longitudinale Komponenten $E_{longitudinal}$, $H_{longitudinal}$ in Ausbreitungsrichtung, d.h. in Richtung eines Blutgefäßverlaufs, stärker als die transversalen Komponenten $E_{transversal}$, $H_{transversal}$, d.h. quer zum Blutgefäßverlauf, sind. Bevorzugt werden daher in dem zu erfassenden Frequenzbereich diejenigen Moden angeregt, welche eine maximale Einkopplung der Mikrowellenleistung in das Blutgefäß ermöglichen. Eine S-Parametermessung eignet sich besonders gut, die Änderungen des elektro-magnetischen Feldes aufgrund der Belastung mit dem Blutgefäß zu messen.

Gemäß einer Ausführungsform betrifft die Erfindung eine Erfassungsvorrichtung, bei der der Netzwerkanalysator ausgelegt ist, den Blutbildparameter basierend auf einer Messung eines Vorwärts-Transmissionsfaktors, auch als $S_{21}$ bezeichnet, und/oder eines Eingangs-Reflexionsfaktors, auch als $S_{11}$ bezeichnet, zu bestimmen.

Vorfeldmessungen haben gezeigt, dass eine Änderung der Zuckerkonzentration signifikante Einflüsse sowohl auf den Vorwärts-Transmissionsfaktors $S_{21}$ als auch auf den Eingangs-Reflexionsfaktors $S_{11}$ hat. Zur Bestimmung der beiden Streuparameter $S_{21}$ und $S_{21}$ kann ein Netzwerkanalysator, beispielsweise ein vektorieller oder ein skalarer Netzwerkanalysator, eingesetzt werden.

Gemäß einer Ausführungsform betrifft die Erfindung eine Erfassungsvorrichtung, bei der die Änderung des elektro-magnetischen Feldes eine Änderung der Ausbreitungscharakteristik des elektro-magnetischen Feldes gegenüber einer Referenzcharakteristik beschreibt.

Wie oben dargestellt, kann die Referenzcharakteristik aus Messungen der Ausbreitungscharakteristik des elektro-magnetischen Feldes in einem Wassergefäß erstellt werden. Das Gefäß kann eine wässrige Lösung enthalten, in dem ein Stoff gelöst ist, der dazu geeignet ist, die charakteristischen Eigenschaften eines spezifischen Blutbildes zu beschreiben, beispielsweise Zucker, Proteine, Salze, Elektrolyte, Nährstoffe oder Hormone. Zusätzlich oder alternativ kann eine Referenzmessung an einem echten Blutgefäß vorgenommen werden, bei dem der zu erfassende Blutbildparameter bekannt ist. Bei dem Blutbildparameter kann es sich beispielsweise um eine Konzentration eines Blutbestandteils, insbesondere Zuckers wie Glucose, oder Laktats oder Milchsäure oder Sauerstoffs, handeln.

Gemäß einer Ausführungsform betrifft die Erfindung eine Erfassungsvorrichtung, bei der die Referenzcharakteristik eine Ausbreitungscharakteristik des elektro-magnetischen Feldes in Wasser beschreibt. Es kann sich um destilliertes Wasser oder um Wasser einer bestimmten Ionenkonzentration handeln.

Gemäß einer Ausführungsform betrifft die Erfindung eine Erfassungsvorrichtung, bei der der Netzwerkanalysator ausgelegt ist, den Blutbildparameter in einem Frequenzbereich von 1 bis 100 GHz zu bestimmen.

Simulationen haben gezeigt, dass eine Blutader als quasi dielektrischer Wellenleiter sehr hohe Verluste aufweist, die Wellenführung an sich aber funktioniert. Der Hintergrund ist die Leitfähigkeit des Blutes. Wegen dieses Effektes steigen auch die Verluste zu hohen Frequenzen an. Dies bedeutet aber andererseits, dass eine Messung im hohen Frequenzbereich sehr genaue Aufschlüsse über die Beschaffenheit des Bluts liefert. So können bei Frequenzen im Gigahertzbereich die Streuparameter besonders genau bestimmt werden, insbesondere haben Simulationen gezeigt, dass Änderungen der Permittivität und der magnetischen Permeabilität in einem höheren Gigahertzbereich von etwa 20 bis etwa 60 GHz deutlich ausgeprägter sind als in einem unteren Gigaherzbereich von 1 bis 15 GHz. Daher eignet sich insbesondere eine Messung im höheren Gigahertzbereich, um den gewünschten Blutbildparameter sehr präzise zu erfassen.

Gemäß einer Ausführungsform betrifft die Erfindung eine Erfassungsvorrichtung, bei der die elektrische Leitungsanordnung eine Länge im Bereich von etwa 1mm bis etwa 20mm aufweist.

Referenzmessungen haben gezeigt, dass sich der Blutbildparameter umso genauer bestimmen lässt, je länger die Leitung ist. Bei längeren Leitungen kann mehr Mikrowellenenergie in das Blutgefäß eingekoppelt werden, wodurch die Änderung des elektro-magnetischen Feldes aufgrund der Belastung durch das Blutgefäß stärker in Ausprägung tritt. Allerdings muss ein Kompromiss zwischen Leitungslänge und Handhabbarkeit der Leitungsanordnung geschlossen werden. Wenn beispielsweise die Leitungsanordnung mittels einer Anpressmanschette auf die Haut gepresst werden soll, ist der Platz für die Leitungsanordnung begrenzt. Ferner verlaufen die Blutgefäße nicht immer geradlinig durch den Körper, so dass bei größeren Längen nicht die gesamte Leitungslänge auf das Blutgefäß einwirken kann. Eine Länge in einem Bereich von etwa 1 mm bis etwa 20 oder 30 mm hat sich bezüglich Handhabbarkeit als vorteilhaft erwiesen.

Gemäß einer Ausführungsform betrifft die Erfindung eine Erfassungsvorrichtung, bei der die elektrischen Leitungsanordnung einen halboffenen Wellenleiter, insbesondere einen geschlitzten Hohlleiter oder eine Mikrostreifenleitung umfasst.

Eine Mikrostreifenleitung besteht aus einem nicht leitenden Substrat, das auf der Unterseite metallisiert ist und das auf der Oberseite einen Leiter in Form eines Streifens aufweist. Die durch den Netzwerkanalysator erzeugte Mikrowellenenergie breitet sich zu einen in dem Zwischenraum zwischen dem Streifenleiter und der Metallisierung aus. Zum anderen treten Feldlinien auch in den freien Raum über dem Streifenleiter ein. Legt man den Streifenleiter an die Haut an, so kann die Mikrowellenenergie mittels der Feldlinien aus dem Streifenleiter austreten, in das Blutgefäß eintreten und wieder zurück in die Leitungsanordnung einkoppeln. Wie bereits oben erwähnt, bildet das Blutgefäß einen Widerstand für die Mikrowellenenergie, welche die Leitungsanordnung belastet und damit die Anordnung der Feldlinien beeinflusst. Statt einer Mikrowellenleitung kann auch ein geschlitzter Hohlleiter verwendet werden, der denselben Effekt bewirkt, wie oben beschrieben. Während Mikrostreifenleitungen sich insbesondere für den Einsatz im Frequenzbereich zwischen einigen hundert Megahertz und etwa 20 Gigahertz eignen, eignen sich Hohlleiter insbesondere für den Einsatz im Zentimeter-Wellenbereich und darunter, d.h. von etwa 3 GHz bis etwa 200 GHz. Bei Frequenzen bis etwa 20 GHz lässt sich somit bevorzugt die Mikrostreifenleitung einsetzen, die einfach und handlich zu fertigen ist, während für den Einsatz von Frequenzen über etwa 20 GHz bevorzugt eine Hohlleiterstruktur zum Einsatz kommen kann, die eine sehr präzise Messung erlaubt. Bei der elektrischen Leitungsanordnung kann es sich insbesondere um eine Erregeranordnung in Form einer Antenne handeln, wie sie in den oben genannten Patentanmeldungen EP 10192470, EP 10192472EP und EP 10192473 näher beschrieben ist, deren Priorität beansprucht wird.

Gemäß einer Ausführungsform betrifft die Erfindung eine Erfassungsvorrichtung, bei der der geschlitzte Hohlleiter einen Rechteckhohlleiter mit einer Mehrzahl kreisförmiger Schlitze oder mit einem rechteckförmigen Schlitz, insbesondere mit einem rechteckförmigen Schlitz der Breite 1 mm und der Länge 20 mm, umfasst.

Rechteckhohlleiter eignen sich insbesondere für den Einsatz im Zentimeter-Wellenbereich und darunter, d.h. von etwa 3 GHz bis etwa 200 GHz und gestatten damit eine sehr präzise Erfassung des Blutbildparameters. Wie bereits oben erläutert, haben Referenzmessungen gezeigt, dass sich der Blutbildparameter umso genauer bestimmen lässt, je länger die Leitung ist. Bei geschlitzten Hohlleitern gelten diese Aussagen entsprechend für die Länge des Schlitzes, durch welchen die Mikrowellenenergie ausgekoppelt wird. Bei längeren Schlitzen kann mehr Mikrowellenenergie in das Blutgefäß eingekoppelt werden, wodurch die Änderung des elektro-magnetischen Feldes aufgrund der Belastung durch das Blutgefäß stärker in Ausprägung tritt. Allerdings muss ein Kompromiss zwischen Schlitzlänge und Handhabbarkeit der Leitungsanordnung geschlossen werden. Da Hohlleiter sehr sperrig sein können, sollte die Schlitzlänge möglichst begrenzt werden. Eine Länge in einem Bereich von etwa 1 mm bis etwa 20 oder 30 mm hat sich bezüglich seiner Handhabbarkeit als vorteilhaft erwiesen.

Gemäß einer Ausführungsform betrifft die Erfindung eine Erfassungsvorrichtung, bei der die Mikrostreifenleitung eine koplanare Mikrostreifenleitung, insbesondere eine masselose koplanare Mikrostreifenleitung mit einer Spaltbreite im Bereich von etwa 0,1 mm bis etwa 0,9 mm umfasst. Referenzmessungen haben gezeigt, dass der Effekt der Feldänderung in diesem Spaltbreitenbereich besonders ausgeprägt ist.

Gemäß einem weiteren Aspekt betrifft die Erfindung ein Verfahren zum Erfassen eines Blutbildparameters von Blut in einem Blutgefäß, mit einem dielektrischen Koppeln einer elektrischen Leitungsanordnung an das Blutgefäß, einem Anregen der elektrischen Leitungsanordnung, so dass ein elektro-magnetisches Feld von der elektrischen Leitungsanordnung ausgeht, einem Erfassen einer Änderung des elektro-magnetischen Feldes bei dielektrischer Belastung der elektrischen Leitungsanordnung mit dem Blutgefäß und einem Erfassen des Blutbildparameters auf der Basis der erfassten Änderung des elektro-magnetischen Feldes.

Weitere Verfahrensschritte ergeben sich unmittelbar aus der Funktionalität der Erfassungsvorrichtung zum Erfassen eines Blutbildparameters.

Weitere Ausführungsbeispiele werden Bezug nehmend auf die beiliegenden Zeichnungen näher erläutert. Es zeigen:

Fig. 1        ein Blockdiagramm einer Erfassungsvorrichtung gemäß einem Ausführungsbeispiel;

Fig. 2        ein Blockdiagramm einer Erfassungsvorrichtung gemäß einem Ausführungsbeispiel;

| | |
|---|---|
| Fig. 3 | eine schematische Darstellung einer Reflexions/Transmissionsmessung zur Erfassung einer Änderung des elektro-magnetischen Feldes in einem Blutgefäß gemäß einem Ausführungsbeispiel; |
| Fig. 4 | eine S-Parameter Darstellung der dielektrischen Kopplung einer Leitungsanordnung mit einem Blutgefäß gemäß einem Ausführungsbeispiel; |
| Fig. 5 | eine grafische Darstellung eines absoluten und eines differentiellen Vorwärts-Transmissionsfaktors $S_{21}$, gemessen mit einer Erfassungsvorrichtung gemäß einem Ausführungsbeispiel, in Abhängigkeit der Frequenz bei verschiedenen Zuckerkonzentrationen in dem Blutgefäß; |
| Fig. 6 | eine grafische Darstellung eines differentiellen Eingangs-Reflexionsfaktors $S_{11}$, gemessen mit einer Erfassungsvorrichtung gemäß einem Ausführungsbeispiel, in Abhängigkeit der Frequenz bei verschiedenen Zuckerkonzentrationen in dem Blutgefäß; |
| Fig. 7 | eine grafische Darstellung einer differentiellen Phase eines Vorwärts-Transmissionsfaktors $S_{21}$, gemessen mit einer Erfassungsvorrichtung gemäß einem Ausführungsbeispiel, in Abhängigkeit der Frequenz bei verschiedenen Leitungslängen der Leitungsanordnung; |
| Fig. 8 | eine grafische Darstellung einer differentiellen Phase eines Vorwärts-Transmissionsfaktors $S_{21}$, gemessen mit einer Erfassungsvorrichtung gemäß einem Ausführungsbeispiel, in Abhängigkeit der Zuckerkonzentration bei verschiedenen Leitungslängen der Leitungsanordnung; |
| Fig. 9 | eine grafische Darstellung einer differentiellen Phase eines Vorwärts-Transmissionsfaktors $S_{21}$, gemessen mit einer Erfassungsvorrichtung gemäß einem Ausführungsbeispiel, in Abhängigkeit der Frequenz bei verschiedenen Zuckerkonzentrationen in dem Blutgefäß; |
| Fig. 10 | eine Leitungsanordnung gemäß einem Ausführungsbeispiel; |
| Fig. 11 | eine grafische Darstellung einer differentiellen Phase eines Vorwärts-Transmissionsfaktors $S_{21}$, gemessen mit der in Fig. 10 dargestellten Leitungsanordnung, in Abhängigkeit der Frequenz bei verschiedenen Leitungsabständen der beiden Leiter der Leitungsanordnung; |
| Fig. 12 | eine Leitungsanordnung gemäß einem Ausführungsbeispiel; |
| Fig. 13 | eine Leitungsanordnung gemäß einem Ausführungsbeispiel; |
| Fig. 14 | eine grafische Darstellung einer differentiellen Phase eines Vorwärts-Transmissionsfaktors $S_{21}$, gemessen mit der in Fig. 13 dargestellten Leitungsanordnung, in Abhängigkeit der Frequenz; |
| Fig. 15 | zwei unterschiedliche Ausführungsformen der in Fig. 13 dargestellten Leitungsanordnung; |
| Fig. 16 | eine schematische Darstellung eines Verfahrens zum Erfassen eines Blutbildparameters von Blut in einem Blutgefäß gemäß einem Ausführungsbeispiel; |
| Fig. 17 | eine Frequenzverschiebung eines Absorptionsmaximums; |
| Fig. 18 | Transmissionsverhalten; |
| Fig. 19 | Frequenzverschiebungen; |
| Fig. 20A-20C | ein Modell eines Querschnitts eines menschlichen Unterarms. |
| Fig. 22 | ein Diagramm zur Illustrierung der reellen Dielektrizitätszahl $\varepsilon'$ und der komplexen Dielektrizitätszahl $\varepsilon''$ in Abhängigkeit der Frequenz; und |
| Fig. 23 | ein Diagramm zur Illustrierung eines Zusammenhangs zwischen der Relaxationszeitkonstanten ($\tau$) und der Glucosekonzentration im Blut. |

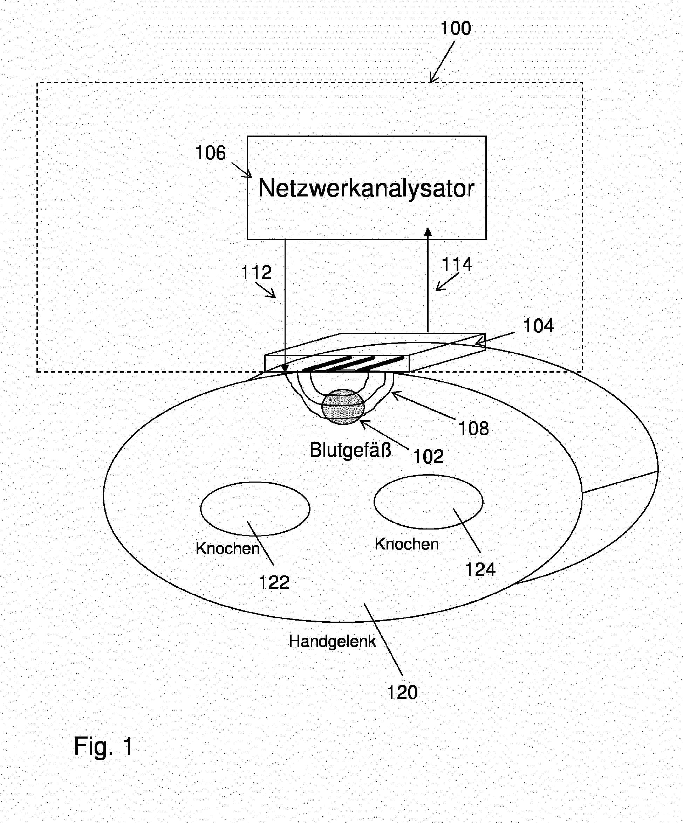

Fig. 1 zeigt ein Blockdiagramm einer Erfassungsvorrichtung 100 zum Erfassen eines Blutbildparameters von Blut in einem Blutgefäß 102, beispielsweise eine Konzentration von Blutzucker in dem Blutgefäß 102, gemäß einem Ausführungsbeispiel. Die Erfassungsvorrichtung 100 umfasst eine elektrische Leitungsanordnung 104, welche an das Blutgefäß 102 dielektrisch koppelbar ist und einen Netzwerkanalysator 106 zum Anregen der Leitungsanordnung 104, so dass ein elektro-magnetisches Feld 108 von der Leitungsanordnung 104 ausgeht.

Die Erfassungsvorrichtung 100 umfasst ferner einen Netzwerkanalysator 106, welcher ausgelegt ist, eine Änderung des elektro-magnetischen Feldes 108 bei dielektrischer Belastung der Leitungsanordnung 104 mit dem Blutgefäß 102 zu erfassen und auf der Basis der erfassten Änderung des elektro-magnetischen Feldes 108 den Blutbildparameter zu erfassen. Die Leitungsanordnung 104 besteht aus mindestens zwei elektrischen Leitungen, die an mindestens einem ersten Port 112 und mindestens einem zweiten Port 114 zugänglich sind, um den Netzwerkanalysator 106 anzuschließen. Die halboffene Leitungsanordnung 104 kann beispielsweise eine koplanare Mikrostreifenleitung oder ein Hohlleiter sein. Die Erfassungseinrichtung 100 kann einen Prozessor aufweisen, mit dem der Blutbildparameter basierend auf der erfassten Änderung des elektro-magnetischen Feldes bestimmt wird. Der Prozessor kann beispielsweise mittels einer Streuparametermessung die Änderung der Feldlinien bestimmen. Die Erfassungsvorrichtung 100 kann ferner einen Speicher aufweisen, in dem Referenzwerte für Abstrahlcharakteristika abgespeichert sind, um basierend auf einem Vergleich mit den Referenzwerten den Blutbildparameter zu bestimmen.

Gemäß einer Ausführungsform ist der Netzwerkanalysator 106 ein Element der Erfassungsvorrichtung 100. Gemäß einer anderen Ausführungsform ist der Netzwerkanalysator 106 kein Element oder ein optionales Element der Erfassungsvorrichtung 100.

Die in Fig. 1 dargestellte Erfassungsvorrichtung 100 nutzt die Erkenntnis aus, dass ein Blutgefäß 102, eine Hautschicht sowie ein das Blutgefäß 102 umgebendes Fettgewebe beispielsweise eines menschlichen Unterarms oder Handgelenks 120 als ein dielektrisches Wellenleitersystem aufgefasst werden können. Der Aufbau eines menschlichen Unterarms ist beispielsweise in Netter, F. N., Atlas der Anatomie, Thieme Verlag, 2006, beschrieben. Ein menschlicher Unterarm besteht aus zwei Knochen 122, 124, welche von einem Muskelgewebe umgeben sind. Um das Muskelgewebe herum sind oberflächliche Venen, d.h. Blutgefäße 102, verteilt. Die Knochen 122, 124, das Muskelgewebe sowie die Venen sind durch ein Fettgewebe ummantelt, welches durch obere Hautschichten bedeckt ist. Die oberflächlichen Venen sind relativ nahe an den oberen Hautschichten angeordnet und von diesen durch das Fettgewebe separiert. Wird beispielsweise die in Fig. 1 dargestellte Leitungsanordnung 104 auf die obere Hautschicht aufgelegt, so kann die Leitungsanordnung 104 dazu eingesetzt werden, in das durch ein Blutgefäß 102, ein Fettgewebe sowie eine Hautschicht gebildete dielektrische Wellenleitersystem beispielsweise eine transversal-elektrische (TE) Welle oder eine transversal-magnetische (TM) Welle einzukoppeln. Dabei können die Hautschicht sowie das Fettgewebe als ein Filmwellenleiter verstanden werden.

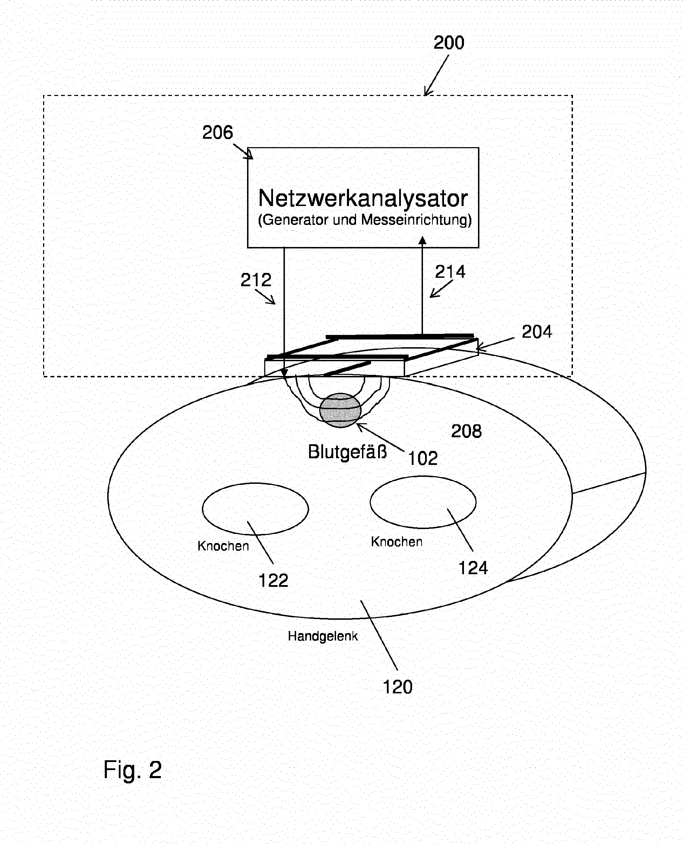

Fig. 2 zeigt ein Blockdiagramm einer Erfassungsvorrichtung 200 zum Erfassen eines Blutbildparameters von Blut in einem Blutgefäß 102, beispielsweise eine Konzentration von Blutzucker in dem Blutgefäß 102, gemäß einem Ausführungsbeispiel. Die Erfassungsvorrichtung 200 umfasst eine elektrische Leitungsanordnung 204, welche an das Blutgefäß 102 dielektrisch koppelbar ist und einen Netzwerkanalysator 206 zum Anregen der Leitungsanordnung 204, so dass ein elektro-magnetisches Feld 208 von der Leitungsanordnung 204 ausgeht. Die Erfassungsvorrichtung 100 umfasst ferner eine Messeinrichtung 210, welche ausgelegt ist, eine Änderung des elektro-magnetischen Feldes 208 bei dielektrischer Belastung der Leitungsanordnung 204 mit dem Blutgefäß 102 zu erfassen und auf der Basis der erfassten Änderung des elektro-magnetischen Feldes 208 den Blutbildparameter zu erfassen. Die Erfassungsvorrichtung 200 unterscheidet sich von der Erfassungsvorrichtung 100 dadurch, dass die Leitungsanordnung 204 als eine Streifenleitung ausgebildet ist. Der Netzwerkanalysator 206 entspricht dem in Fig. 1 dargestellten Netzwerkanalysator 106.

Die Streifenleitung 204 bzw. Streifenleiterstruktur besteht aus einem oder mehreren dünnen, leitfähigen Streifen, die auf einem Dielektrikum aufgebracht sind. Die Streifenleiterstruktur 204 besteht aus in einer Ebene angeordneten Leitungsstreifen, wobei in Fig. 2 zwei dieser Streifen sichtbar sind, die isoliert unter einer metallischen Fläche angeordnet sind. Die Streifenleitung 204 ist auf die Hautoberfläche ausgerichtet, d.h., die beiden Streifen berühren die Haut, während die metallische Grundfläche von der Haut wegweist. Bei Anregung der Streifenleitung 204 mit dem Netzwerkanalysator 206 tritt Mikrowellenenergie über die Feldlinien 208 aus den Streifen in das Handgelenk 120 und wird im Blutgefäß 102 konzentriert. Die Feldlinien schließen sich über das Dielektrikum des Streifenleiters 204.

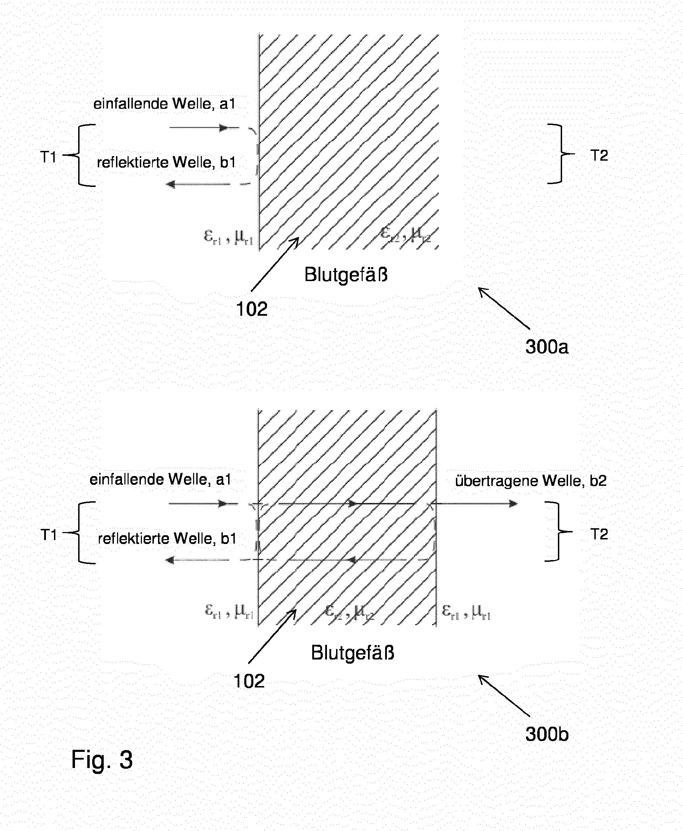

Fig. 3 zeigt eine schematische Darstellung einer Reflexions- 300a / Transmissionsmessung 300b zur Erfassung einer Änderung des elektro-magnetischen Feldes in einem Blutgefäß 102 gemäß einem Ausführungsbeispiel. Das Blutgefäß 102 lässt sich durch seine dielektrische Leitfähigkeit $\varepsilon_{r2}$ und seine magnetische Leitfähigkeit $\mu_{r2}$ charakterisieren. Diese Parameter unterscheiden sich von der dielektrischen Leitfähigkeit $\varepsilon_{r1}$ und der magnetischen Leitfähigkeit $\mu_{r1}$ außerhalb des Blutgefäßes 102. Das Blutgefäß 102 kann als ein Zweitor modelliert werden und mittels Streuparametermessungen vermessen werden.

Bei der Reflexionsmessung 300a wird an einem ersten Tor T1 eine einfallende Welle a1 in Richtung des Blutgefäßes 102 abgestrahlt. Bei Einfall einer elektro-magnetischen Welle a1 auf die Wand des Blutgefäßes 102 wird die Welle aufgrund der Unterschiede in den elektrischen und magnetischen Leitfähigkeiten von dem Blutgefäß 102 reflektiert und kann als reflektierte Welle b1 am Tor T1 gemessen werden. Am Tor T2 findet keine Aktivität statt.

Bei der Transmissionsmessung 300b wird an dem ersten Tor T1 die einfallende Welle a1 in Richtung des Blutgefäßes 102 abgestrahlt. Bei Einfall einer elektro-magnetischen Welle a1 auf die Wand des Blutgefäßes 102 wird die Welle aufgrund der Unterschiede in den elektrischen und magnetischen Leitfähigkeiten teilweise von dem Blutgefäß 102 reflektiert. Ein Teil der Energie wird als reflektierte Welle b1 am Tor T1 reflektiert und kann dort gemessen werden. Ein weiterer Teil der Energie durchdringt das Blutgefäß 102 und kann als übertragene Welle b2 am Tor T2 gemessen werden.

Bei der Modellierung des Blutgefäßes mittels Zweitordarstellung umfassen die S-Parameter die Elemente $S_{11}$, $S_{12}$, $S_{21}$ und $S_{22}$:

$$\begin{pmatrix} b_1 \\ b_2 \end{pmatrix} = \begin{pmatrix} S_{11} & S_{12} \\ S_{21} & S_{22} \end{pmatrix} \begin{pmatrix} a_1 \\ a_2 \end{pmatrix}$$

$a_1$ ist die am Tor 1 einlaufende Welle, $a_2$ die am Tor 2 einlaufende Welle. $b_1$ beschreibt die vom Eingang (Tor 1) auslaufende Welle, $b_2$ beschreibt die vom Ausgang (Tor 2) auslaufende Welle.

Die S-Parameter haben dabei folgende Bedeutung:

Der Eingangsreflexionsfaktor $S_{11}$

$$S_{11} = \left.\frac{b_1}{a_1}\right|_{a_2=0}$$

stellt die Reflexion am Eingang ohne Welleneinprägung am Tor 2 dar.
Der Ausgangsreflexionsfaktor $S_{22}$

$$S_{22} = \left.\frac{b_2}{a_2}\right|_{a_1=0}$$

stellt die Reflexion am Tor 2 ohne Anregung an Tor 1 dar.
Der Vorwärts-Transmissionsfaktor $S_{21}$

$$S_{21} = \left.\frac{b_2}{a_1}\right|_{a_2=0}$$

stellt die Vorwärts-Transmission ohne Anregung am Tor 2 dar.
Der Rückwärts-Transmissionsfaktor $S_{12}$

$$S_{12} = \left.\frac{b_1}{a_2}\right|_{a_1=0}$$

stellt die Rückwärts-Transmission ohne Anregung am Tor 1 dar.

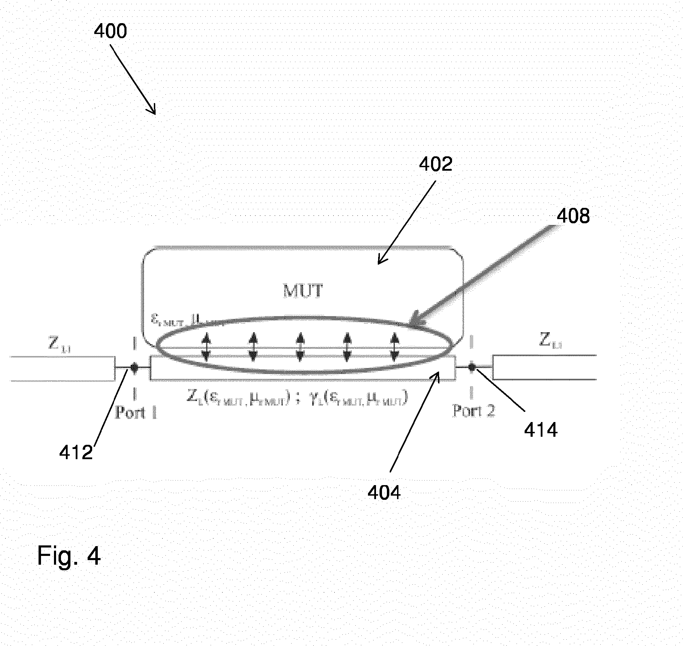

Fig. 4 zeigt eine S-Parameter Darstellung 400 der dielektrischen Kopplung einer Leitungsanordnung mit einem Blutgefäß gemäß einem Ausführungsbeispiel. Dabei wird eine Leitungsanordnung 404 an den beiden Ports Port 1, 412 und Port 2, 414 mit Mikrowellenenergie angeregt. Die Ports 412 und 414 entsprechen dabei den in Fig. 1 dargestellten Ports 112 und 114, über die der Netzwerkanalysator 106 die Leitungsanordnung anregt und die Änderung des elektromagnetischen Feldes 108 erfasst. Die beiden Impedanzen $Z_{L1}$ links und rechts der beiden Ports 412, 414 stellen beispielsweise Innenwiderstände des Netzwerkanalysators oder der Messeinrichtung dar, während die Impedanz $Z_L$ ($\varepsilon_{r,MUT}$, $\mu_{r,MUT}$) zwischen den beiden Ports 412, 414 den Wellenwiderstand der mit dem Blutgefäß 402 belasteten Leitungsanordnung 404 bezeichnet. Das Blutgefäß 402 wird hier auch durch die Bezeichnung "MUT, material under test" spezifiziert. Bei der Ausbreitung von elektromagnetischen Wellen 408 auf der Leitungsanordnung 404 entsteht am Leitungsende eine Reflexion, wenn die dort angeschlossene Schaltung eine vom Wert der Wellenimpedanz $Z_L(\varepsilon_{r1}, \mu_{r1})$ der unbelasteten Leitungsanordnung 404 abweichende Eingangsimpedanz $Z_L(\varepsilon_{r2}, \mu_{r2})$ besitzt. Das Verhältnis von reflektierter zu hinlaufender Spannungswelle wird als *Reflexionsfaktor* bezeichnet und wird nach folgender Gleichung berechnet:

$$ r = \frac{\vec{E}_{2tan}^{-}}{\vec{E}_{1tan}^{+}} = \frac{Z_2(\epsilon_{r2}, \mu_{r2}) - Z_1(\epsilon_{r1}, \mu_{r1})}{Z_2(\epsilon_{r2}, \mu_{r2}) + Z_1(\epsilon_{r1}, \mu_{r1})} $$

Dabei bedeuten

- $Z_L(\varepsilon_{r1}, \mu_{r1})$: der Wellenwiderstand der unbelasteten Leitungsanordnung,
- $Z_L(\varepsilon_{r2}, \mu_{r2})$: der Eingangswiderstand des an die Leitungsanordnung 404 gekoppelten Blutgefäßes 402,
- $E_{1tan}$: die Feldstärke der hinlaufenden Welle
- $E_{2tan}$: die Feldstärke der rücklaufenden Welle

Für $Z_L(\varepsilon_{r1}, \mu_{r1}) = Z_L(\varepsilon_{r2}, \mu_{r2})$ wird der Reflexionsfaktor zu Null. Dieser Zustand wird angestrebt, weil dann die maximale Leistung übertragen wird und die Messwertauflösung am größten ist, um den Blutbildparameter zu bestimmen. Man spricht dann von Impedanzanpassung oder Leistungsanpassung.

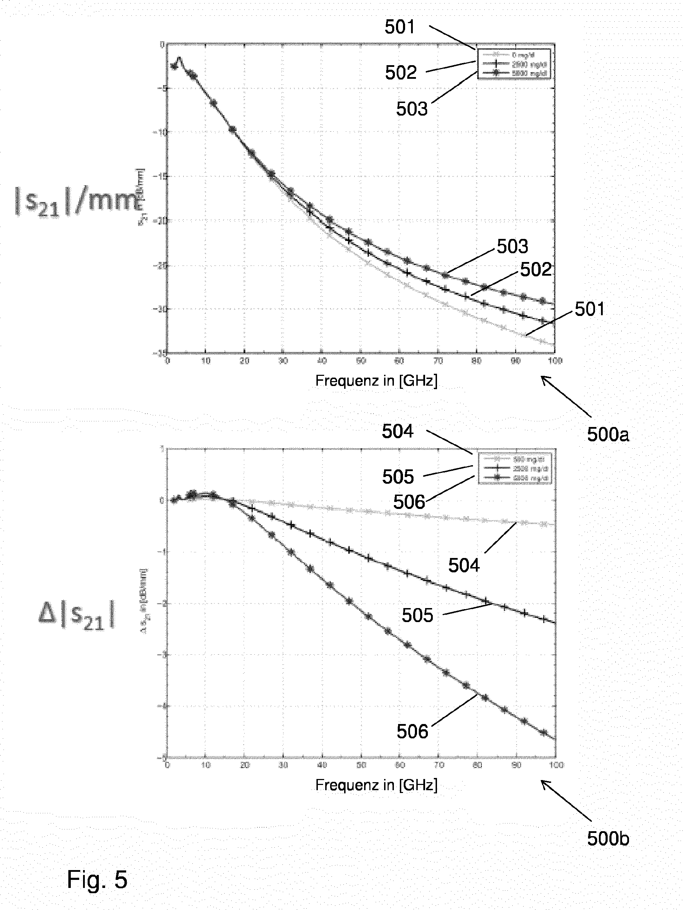

Fig. 5 zeigt eine grafische Darstellung eines absoluten und eines differentiellen Vorwärts-Transmissionsfaktors $S_{21}$, gemessen mit einer Erfassungsvorrichtung gemäß einem Ausführungsbeispiel, in Abhängigkeit der Frequenz bei verschiedenen Zuckerkonzentrationen in dem Blutgefäß. Die obere Darstellung 500a zeigt den Betrag des absoluten Vorwärts-Transmissionsfaktors $S_{21}$ in einem Frequenzbereich von 0 bis 100 GHz. Dabei zeigt eine erste Kurve 501 den Betragsverlauf des Vorwärts-Transmissionsfaktors $|S_{21}|$ bei einer Zuckerkonzentration von 0 mg/dl, eine zweite Kurve 502 den Betragsverlauf des Vorwärts-Transmissionsfaktors $|S_{21}|$ bei einer Zuckerkonzentration von 2500 mg/dl und eine dritte Kurve 503 den Betragsverlauf des Vorwärts-Transmissionsfaktors $|S_{21}|$ bei einer Zuckerkonzentration von 5000 mg/dl.

Die untere Darstellung 500b zeigt den Verlauf des differentiellen Vorwärts-Transmissionsfaktors $S_{21}$ als Differenz des absoluten Vorwärts-Transmissionsfaktors zu einem Referenzwert, der hier dem Verlauf 501 entspricht, d.h. dem Verlauf des absoluten Vorwärts-Transmissionsfaktors bei einer Zuckerkonzentration von 0mg/dl, in einem Frequenzbereich von 0 bis 100 GHz. Dabei zeigt eine erste Kurve 504 den Verlauf des differentiellen Vorwärts-Transmissionsfaktors $\Delta|S_{21}|$ bei einer Zuckerkonzentration von 500 mg/dl, eine zweite Kurve 505 den Verlauf des differentiellen Vorwärts-Transmissionsfaktors $\Delta|S_{21}|$ bei einer Zuckerkonzentration von 2500 mg/dl und eine dritte Kurve 506 den Verlauf des differentiellen Vorwärts-Transmissionsfaktors $\Delta|S_{21}|$ bei einer Zuckerkonzentration von 5000 mg/dl. Je höher die Zuckerkonzentration, desto bessere Auflösung zeigt der differentielle Vorwärts-Transmissionsfaktors $\Delta|S_{21}|$.

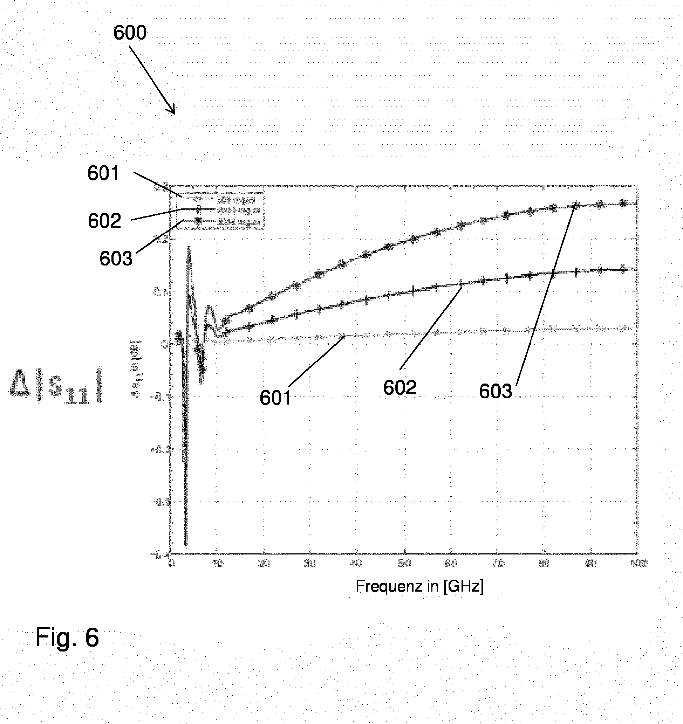

Fig. 6 zeigt eine grafische Darstellung 600 eines differentiellen Eingangs-Reflexionsfaktors $S_{11}$, gemessen mit einer Erfassungsvorrichtung gemäß einem Ausführungsbeispiel, in Abhängigkeit der Frequenz bei verschiedenen Zuckerkonzentrationen in dem Blutgefäß.

Der differentielle Eingangs-Reflexionsfaktor $\Delta|S_{11}|$ ist als Differenz des absoluten differentielle Eingangs-Reflexionsfaktors zu einem Referenzwert, der dem Verlauf des absoluten Eingangs-Reflexionsfaktors bei einer Zuckerkonzentration von 0mg/dl entspricht, in einem Frequenzbereich von 0 bis 100 GHz dargestellt. Dabei zeigt eine erste Kurve 601 den Verlauf des differentiellen Eingangs-Reflexionsfaktors $\Delta|S_{11}|$ bei einer Zuckerkonzentration von 500 mg/dl, eine zweite Kurve 602 den Verlauf des differentiellen Eingangs-Reflexionsfaktors $\Delta|S_{11}|$ bei einer Zuckerkonzentration von 2500 mg/dl und eine dritte Kurve 603 den Verlauf des differentiellen Eingangs-Reflexionsfaktors $\Delta|S_{11}|$ bei einer Zuckerkonzentration von 5000 mg/dl. Je höher die Zuckerkonzentration, desto besser ist die Auflösung des differentiellen Eingangs-Reflexionsfaktors $\Delta|S_{11}|$.

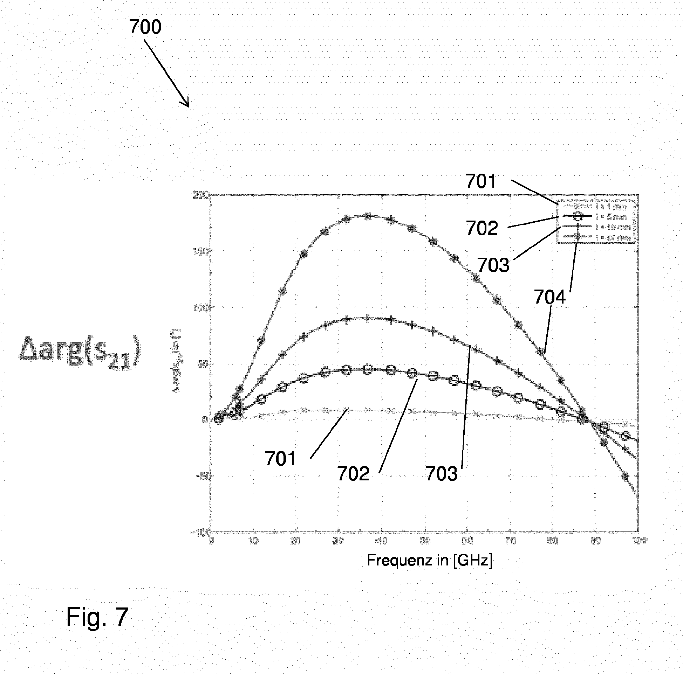

Fig. 7 zeigt eine grafische Darstellung 700 einer differentiellen Phase eines Vorwärts-Transmissionsfaktors $S_{21}$, gemessen mit einer Erfassungsvorrichtung gemäß einem Ausführungsbeispiel, gemessen über eine Frequenz im Bereich von 0 bis 100 GHz bei verschiedenen Leitungslängen der Leitungsanordnung. Die differentielle Phase bestimmt sich als Differenz der Phase eines Referenz-Vorwärts-Transmissionsfaktors $S_{21}$ zu der Phase eines gemessenen Vorwärts-Transmissionsfaktors $S_{21}$.

Dabei zeigt eine erste Kurve 701 den Verlauf der differentiellen Phase des Vorwärts-Transmissionsfaktors $\Delta arg|S_{21}|$ bei einer Leitungslänge von 1 mm, eine zweite Kurve 702 den Verlauf der differentiellen Phase des Vorwärts-Transmissionsfaktors $\Delta arg|S_{21}|$ bei einer Leitungslänge von 5 mm, eine dritte Kurve 703 den Verlauf der differentiellen Phase des Vorwärts-Transmissionsfaktors $\Delta arg|S_{21}|$ bei einer Leitungslänge von 10 mm und eine vierte Kurve 704 den Verlauf der differentiellen Phase des Vorwärts-Transmissionsfaktors $\Delta arg|S_{21}|$ bei einer Leitungslänge von 20 mm. Je größer die Leitungslänge, desto bessere Auflösung zeigt die differentielle Phase des Vorwärts-Transmissionsfaktors $\Delta arg|S_{21}|$.

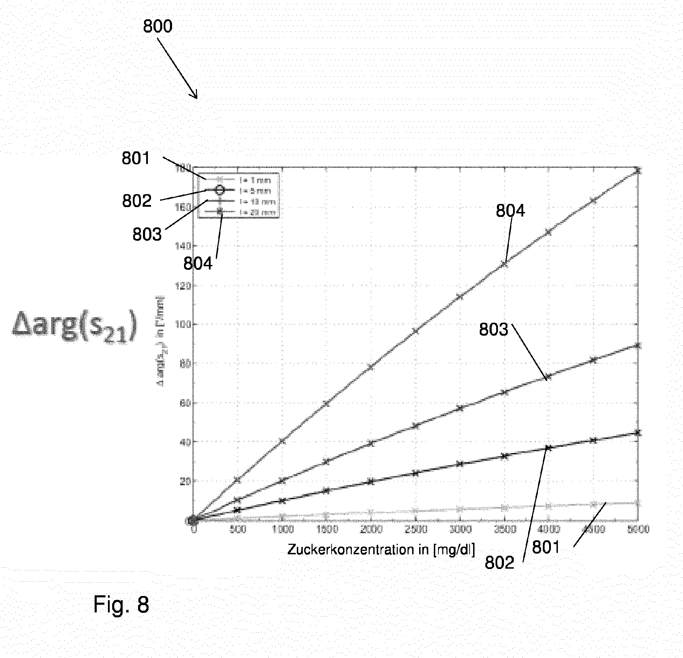

Fig. 8 zeigt eine grafische Darstellung 800 einer differentiellen Phase eines Vorwärts-Transmissionsfaktors $S_{21}$, gemessen mit einer Erfassungsvorrichtung gemäß einem Ausführungsbeispiel, in Abhängigkeit der Zuckerkonzentration, gemessen über den Bereich von 0 bis 500 mg/dl bei verschiedenen Leitungslängen der Leitungsanordnung.

Dabei zeigt eine erste Kurve 801 den Verlauf der differentiellen Phase des Vorwärts-Transmissionsfaktors $\Delta arg|S_{21}|$ bei einer Leitungslänge von 1 mm, eine zweite Kurve 802 den Verlauf der differentiellen Phase des Vorwärts-Transmissionsfaktors $\Delta arg|S_{21}|$ bei einer Leitungslänge von 5 mm, eine dritte Kurve 803 den Verlauf der differentiellen Phase des Vorwärts-Transmissionsfaktors $\Delta arg|S_{21}|$ bei einer Leitungslänge von 10 mm und eine vierte Kurve 804 den Verlauf der differentiellen Phase des Vorwärts-Transmissionsfaktors $\Delta arg|S_{21}|$ bei einer Leitungslänge von 20 mm. Je größer die Leitungslänge, desto bessere Auflösung zeigt die differentielle Phase des Vorwärts-Transmissionsfaktors $\Delta arg|S_{21}|$. Ferner gilt: Je größer die Zuckerkonzentration, desto bessere Auflösung zeigt die differentielle Phase des Vorwärts-Transmissionsfaktors $\Delta arg|S_{21}|$.

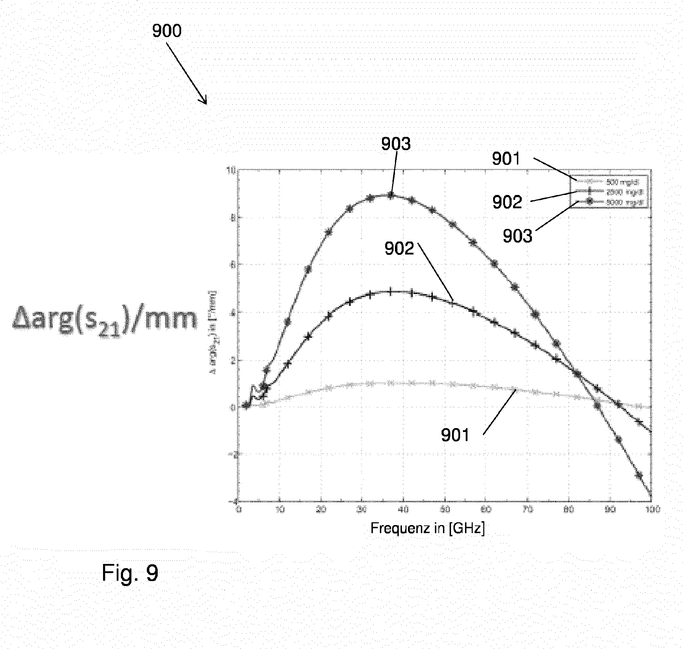

Fig. 9 zeigt eine grafische Darstellung 900 einer differentiellen Phase eines Vorwärts-Transmissionsfaktors $S_{21}$, gemessen mit einer Erfassungsvorrichtung gemäß einem Ausführungsbeispiel, in Abhängigkeit der Frequenz bei verschiedenen Zuckerkonzentrationen in dem Blutgefäß.

Dabei zeigt eine erste Kurve 901 den Verlauf der differentiellen Phase des Vorwärts-Transmissionsfaktors $\Delta arg|S_{21}|$ bei einer Zuckerkonzentration von 500 mg/dl, eine zweite Kurve 902 den Verlauf der differentiellen Phase des Vorwärts-Transmissionsfaktors $\Delta arg|S_{21}|$ bei einer Zuckerkonzentration von 2500 mg/dl und eine dritte Kurve 903 den Verlauf der differentiellen Phase des Vorwärts-Transmissionsfaktors $\Delta arg|S_{21}|$ bei einer Zuckerkonzentration von 5000 mg/dl. Je größer die Zuckerkonzentration, desto bessere Auflösung zeigt die differentielle Phase des Vorwärts-Transmissionsfaktors $\Delta arg|S_{21}|$.

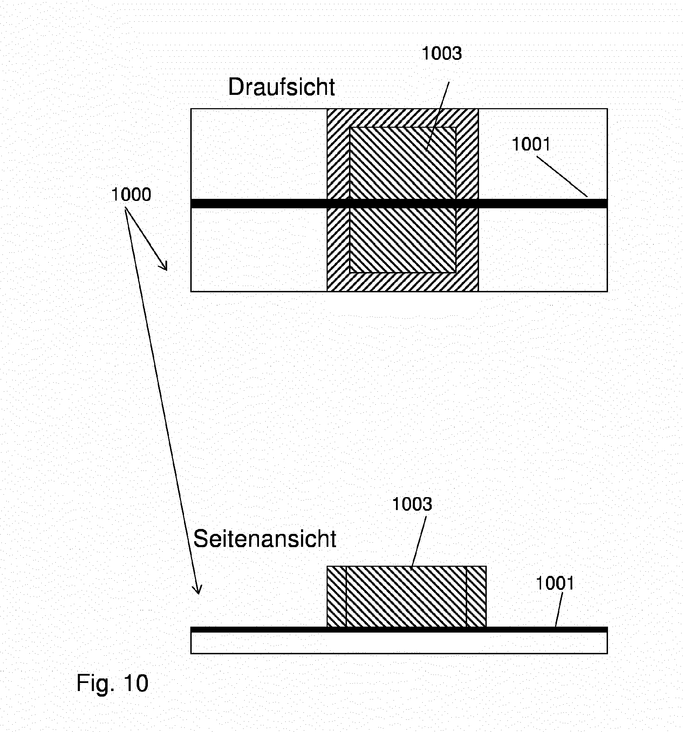

Fig. 10 zeigt eine Leitungsanordnung 1000 gemäß einem Ausführungsbeispiel. Bei der Leitungsanordnung 1000 handelt es sich um eine Mikrostreifenleitung. Die Mikrostreifenleitung 1000 besteht aus einem leitfähigen Streifen 1001, der durch ein dielektrisches Substrat von einer leitfähigen Fläche getrennt ist. Die Mikrostreifenleitung 1000 besteht aus einem nicht leitenden Substrat bzw. einer Leiterplatte, die auf der Unterseite vollständig metallisiert ist, so dass die Metallisierung als Massefläche dient. Auf der Oberseite ist ein Leiter 1001 in Form eines Streifens bzw. einer Leiterbahn, also mit definierter Querschnittsfläche, angeordnet. Dieser Streifen 1001 kann gewöhnlich durch Bearbeitung der oberen Metallisierung durch Ätzen oder Fräsen angefertigt werden. Als Substrat können verschiedene Dielektrika verwendet werden, beispielsweise glasfaserverstärktes PTFE (Polytetrafluorethylen), Aluminiumoxid oder ein anderes keramisches Material. Das von der Leitungsanordnung 1000 abgestrahlte Signal breitet sich zum Einen in dem Zwischenraum zwischen dem Streifenleiter 1001 und der Massefläche aus. Zum Anderen treten die Feldlinien auch in den freien Raum über dem Streifenleiter 1001 ein, der in der Regel mit Luft gefüllt ist. Ein Hohlleiter 1003 ist über dem Streifenleiter 1001 angeordnet, um die Leitungsanordnung 1000 anzuregen.

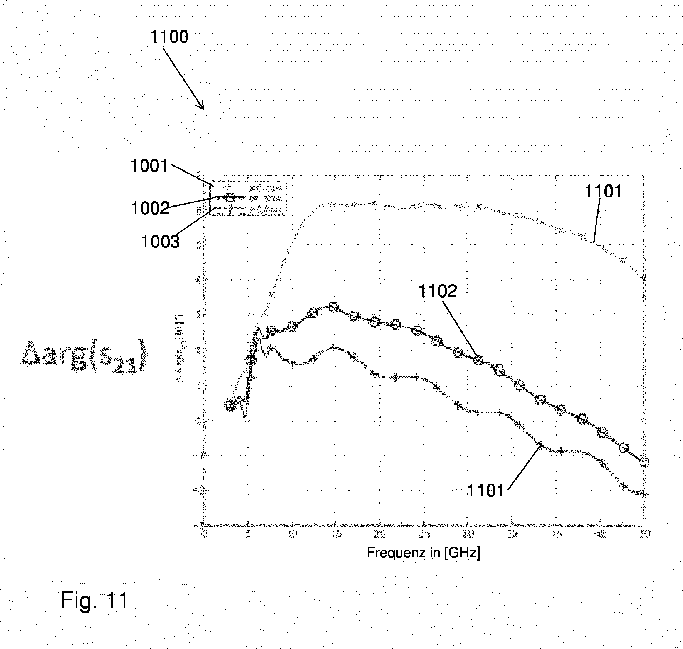

Fig. 11 zeigt eine grafische Darstellung 1100 einer differentiellen Phase eines Vorwärts-Transmissionsfaktors $S_{21}$, gemessen mit der in Fig. 10 dargestellten Leitungsanordnung, in Abhängigkeit der Frequenz bei verschiedenen Leitungsabständen der beiden Leiter der Leitungsanordnung, bzw. bei unterschiedlicher Dicke der Dielektrikumsschicht der Mikrostreifenleitung .

Dabei zeigt eine erste Kurve 1101 den Verlauf der differentiellen Phase des Vorwärts-Transmissionsfaktors $\Delta arg|S_{21}|$ bei einem Leitungsabstand von 0,1 mm, eine zweite Kurve 1102 den Verlauf der differentiellen Phase des Vorwärts-Transmissionsfaktors $\Delta arg|S_{21}|$ bei einem Leitungsabstand von 0,5 mm und eine dritte Kurve 1103 den Verlauf der differentiellen Phase des Vorwärts-Transmissionsfaktors $\Delta arg|S_{21}|$ bei einem Leitungsabstand von 0,9 mm. Je größer der Leitungsabstand zwischen den beiden Leitern, desto bessere Auflösung zeigt die differentielle Phase des Vorwärts-Transmissionsfaktors $\Delta arg|S_{21}|$.

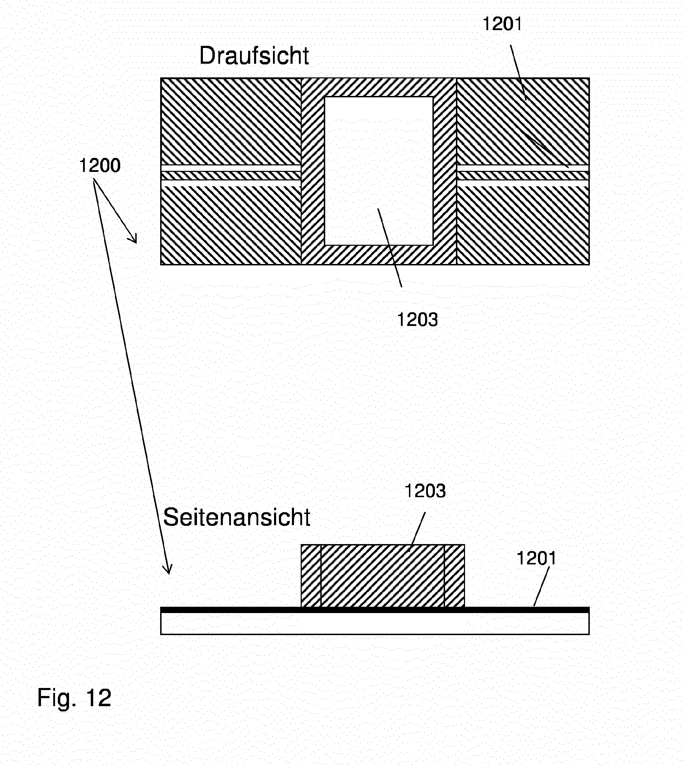

Fig. 12 zeigt eine Leitungsanordnung 1200 gemäß einem Ausführungsbeispiel. Bei der Leitungsanordnung 1200 handelt es sich um einen koplanaren Wellenleiter ohne Massefläche. Der koplanare Wellenleiter 1200 weist einen Spalt 1201 zwischen zwei Metallisierungsflächen auf, der sich über die gesamte Oberseite des Wellenleiters 1200 erstreckt und die beiden Metallisierungsflächen voneinander trennt. Über dem Spalt 1201 ist ein Hohlleiter 1203 zur Einkopplung von Mikrowellenenergie in den koplanaren Wellenleiter 1200 angebracht.

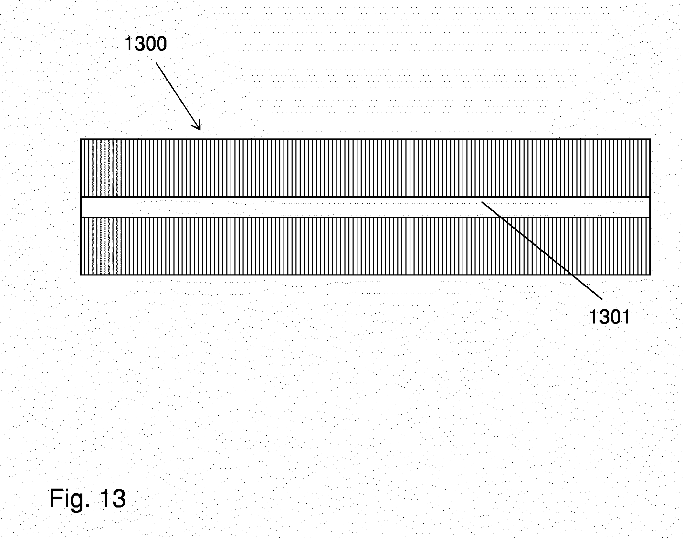

Fig. 13 zeigt eine Leitungsanordnung gemäß einem Ausführungsbeispiel. Bei der Leitungsanordnung 1300 handelt es sich um einen geschlitzten rechteckigen Wellenleiter. An einer Oberseite des Wellenleiters 1300 ist ein Schlitz 1301 angebracht, in den ein Netzwerkanalysator Mikrowellenenergie einkoppeln kann.

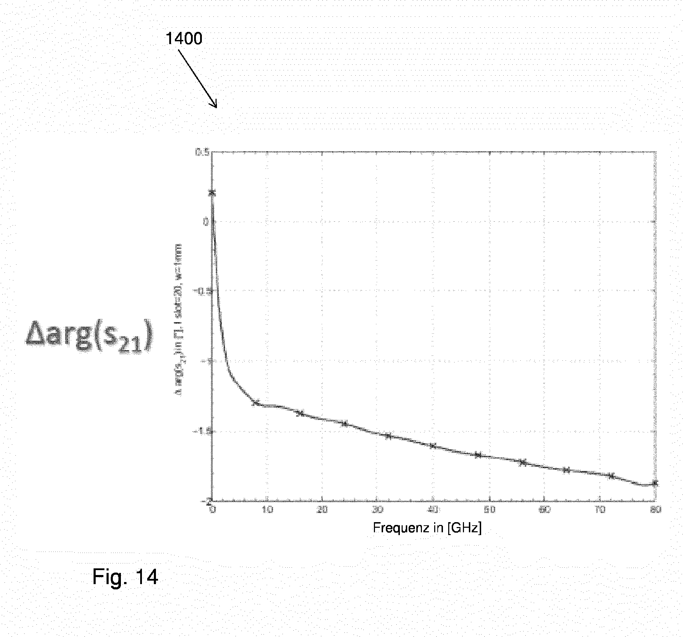

Fig. 14 zeigt eine grafische Darstellung einer Phase eines Vorwärts-Transmissionsfaktors $S_{21}$, gemessen mit der in Fig. 13 dargestellten Leitungsanordnung, in Abhängigkeit der Frequenz, wobei ein Frequenzbereich von 0 bis 80 GHz dargestellt ist. Je größer die Frequenz, desto besser ist die Auflösung der differentiellen Phase des Vorwärts-Transmissionsfaktors $S_{21}$.

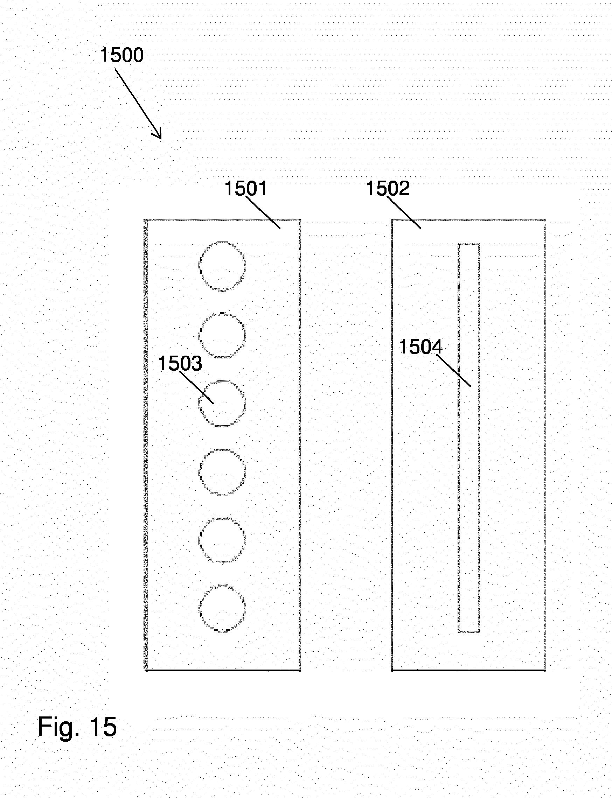

Fig. 15 zeigt zwei unterschiedliche Ausführungsformen der in Fig. 13 dargestellten Leitungsanordnung. Bei einer ersten Ausführungsform 1501, die in der linken Bildhälfte dargestellt ist, weist der rechteckige Wellenleiter eine Anzahl von 6 kreisrunden Schlitzen 1503 auf, wobei statt der Zahl 6 auch jede andere angemessene ganzzahlige Zahl gewählt werden kann. Bei einer zweiten Ausführungsform 1502, die in der rechten Bildhälfte dargestellt ist, weist der rechteckige Wellenleiter einen rechteckigen Schlitz 1504 auf, wobei einem einzigen Schlitz auch mehrere hintereinander angeordnete rechteckförmige Schlitze verwendet werden können.

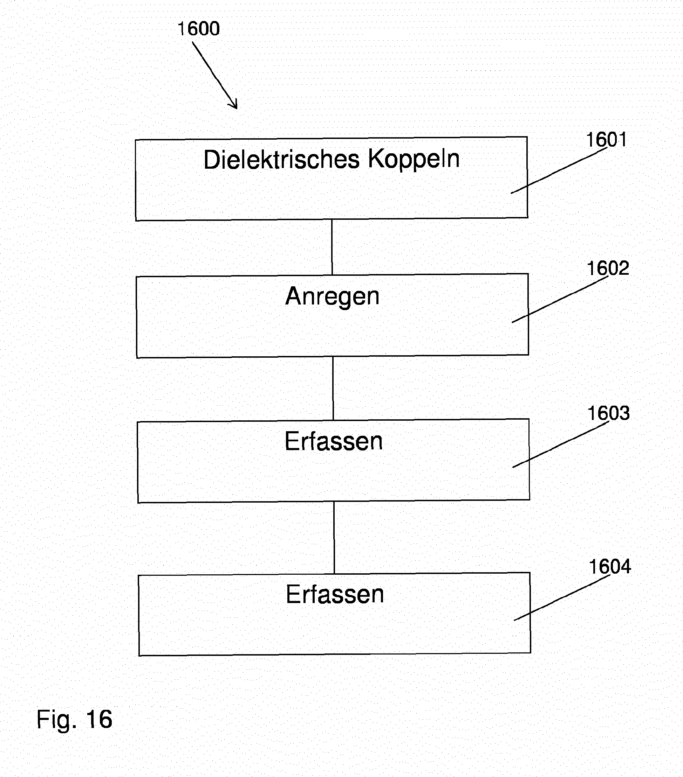

Fig. 16 zeigt eine schematische Darstellung eines Verfahrens 1600 zum Erfassen eines Blutbildparameters von Blut in einem Blutgefäß gemäß einem Ausführungsbeispiel. Das Verfahren 1600 umfasst ein dielektrisches Koppeln 1601 einer elektrischen Leitungsanordnung an das Blutgefäß, ein Anregen 1602 der elektrischen Leitungsanordnung, so dass ein elektro-magnetisches Feld von der elektrischen Leitungsanordnung ausgeht, ein Erfassen 1603 einer Änderung des elektro-magnetischen Feldes bei dielektrischer Belastung der elektrischen Leitungsanordnung mit dem Blutgefäß und ein Erfassen 1604 des Blutbildparameters auf der Basis der erfassten Änderung des elektro-magnetischen Feldes.

Die Messung von $|S_{21}|$ kann wie vorstehend beschrieben ausgeführt werden. Optional kann der in Fig. 1 dargestellte Netzwerkanalysator 207 einen durchstimmbaren Oszillator umfassen.

Gemäß einem weiteren Ausführungsbeispiel kann durch eine weitere Messung eines Betrags des Messparameters von S11 die Genauigkeit bei der Bestimmung der Verlustgrößen noch weiter gesteigert werden. Die Verlustgrößen können beispielsweise auf der Basis der folgenden Formel bestimmt werden:

$$P_{Verlust} = 1 - \left|S_{11}\right|^2 - \left|S_{21}\right|^2,$$

wobei $P_{Verlust}$ die jeweilige Verlustgröße bezeichnet, und wobei $S_{11}$ den Eingangsreflexionsfaktor und $S_{21}$ den Vorwärtstransmissionsfaktor bezeichnen.

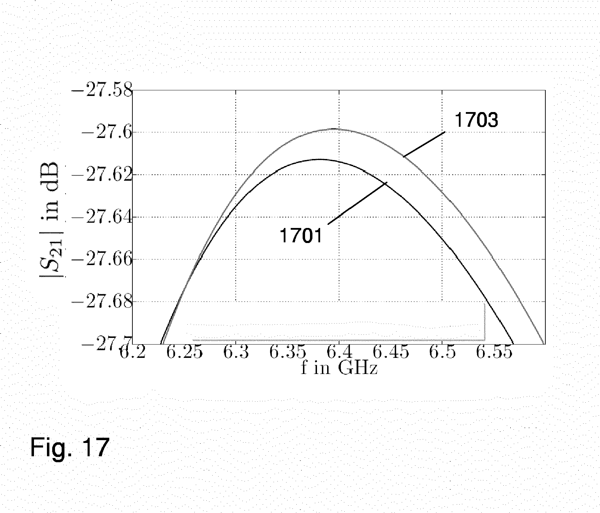

Zum Erfassen des Blutbildparameters, beispielsweise einer Konzentration von Blutzucker, können beispielsweise Frequenzverschiebungen von Absorptionsmaxima einer Wasserlösung mit Zucker untersucht werden. Fig. 17 zeigt beispielsweise eine Frequenzverschiebung eines Absorptionsmaximums 1701 bei einer ersten Blutzuckerkonzentration im Vergleich zu einer Frequenzverschiebung eines Absorptionsmaximums 1703 bei einer zweiten Blutzuckerkonzentration, welche höher als die erste Blutzuckerkonzentration. Dabei wurde als Verlustgröße beispielhaft eine Transmission um 6 GHz erfasst.

Die Frequenzverschiebung des Absorptionsmaximums kann als ein Maß für einen Blutbildparameter, beispielsweise für einen Blutzuckerspiegel, aufgefasst werden. Durch eine Beobachtung von Frequenzverschiebungen bei mehreren Absorptionen einer Wasserlösung mit Zucker kann die Messzuverlässigkeit noch weiter erhöht werden.

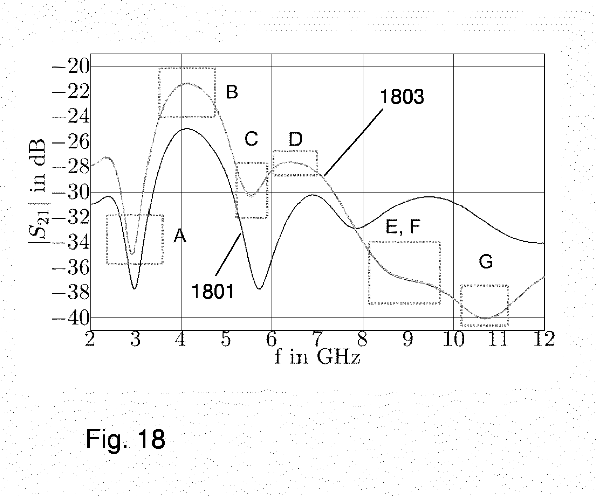

Fig. 18 zeigt Transmissionsverhalten von venösem Blut in einem Handgelenk. Dabei verdeutlichen die Verläufe 1801 und 1803 unterschiedliche Frequenzlagen von Absorptionslinien bei unterschiedlichen Blutzuckerkonzentrationen. Zur Erfassung des Blutbildparameters, wie beispielsweise der Konzentration des Blutzuckers, können beispielsweise gezielt Frequenzverschiebungen der Absorptionen A, B, C, D, E, F und G erfasst werden. So kann beispielsweise für jede Frequenz eines Absorptionsmaximums und/oder eines Absorptionsminimums eine Verschiebung in Richtung höherer oder niedriger Frequenzen je nach Blutzuckerspiegel beispielsweise in einem Frequenzbereich zwischen 2 GHz und 12 GHz beobachtet werden.

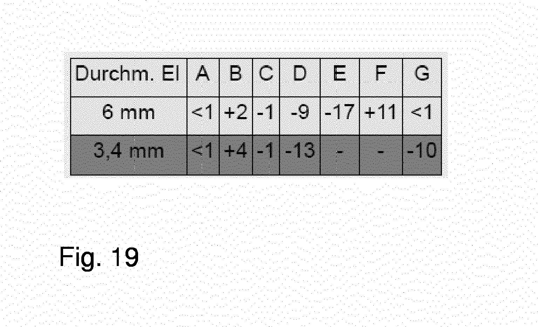

Fig. 19 zeigt beispielhaft Frequenzverschiebungen der in Fig. 17 dargestellten Absorptionen A, B, C, D, E, F und G für ein Blutgefäß mit 6 mm Durchmesser und für ein Blutgefäß mit 3,4 mm Durchmesser. Zu erkennen ist, dass die Absorptionen für eine Zuckerspiegelvariation Frequenzverschiebungen sowohl in positiver als auch in negativer Richtung aufweisen können. Die Erfassung von mehreren Absorptionen bzw. Absorptionslinien ermöglicht somit eine genauere Erfassung eines Blutbildparameters, beispielsweise des Blutzuckerspiegels.

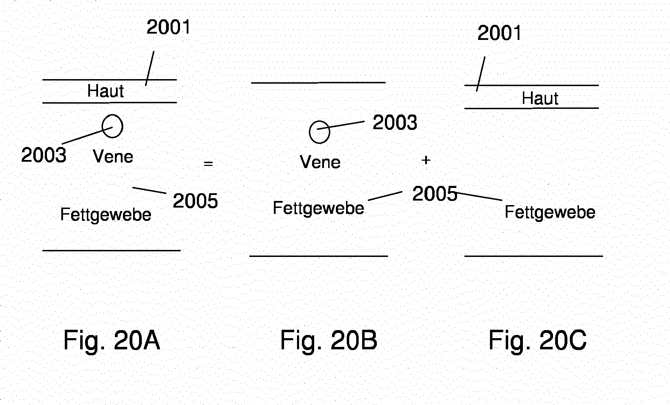

In den Figuren 20A bis 20C ist ein vereinfachtes Modell eines Querschnitts eines menschlichen Unterarms, z.B. eines Handgelenks, wie es beispielsweise für Feldsimulationen bzw. zur Modellierung eines dielektrischen Wellenleitersystems herangezogen werden kann. Wie in Fig. 20A dargestellt, umfasst das Modell eine Hautschicht 2001, ein Blutgefäß 2003 sowie ein das Blutgefäß 2003 umgebendes Fettgewebe 2005. Das in Fig. 20A dargestellte Modell bildet ein dielektrisches Wellenleitersystem umfassend den in Fig. 20B dargestellten dielektrischen Wellenleiter sowie den in Fig. 20C dargestellten elektrischen Filmwellenleiter.

Der in Fig. 20B dargestellte dielektrische Wellenleiter umfasst das Blutgefäß 2003 sowie das dieses umgebende Fettgewebe 2005. Der dielektrische Filmwellenleiter aus Fig. 2C umfasst die Hautschicht 2001 sowie das Fettgewebe 2005. Für die Hautschicht 2001, das Fettgewebe 2005 sowie für das Blutgefäß 2003 kann jeweils ein unterschiedliches dispersives, d.h. frequenzabhängiges, Verhalten der jeweiligen komplexen Dielektrizitätszahl angesetzt werden. Das oben liegende Blutgefäß 2003 wird dabei als ein dielektrischer Wellenleiter interpretiert, in dem je nach Frequenz verschiedene Moden bzw. Wellentypen, beispielsweise eine TE-Welle, eine TM-Welle, eine TEM-Welle oder eine HE-Welle, ausbreitungsfähig sein können. Zu dem Wellenleitermechanismus in dem dielektrischen Wellenleiter kommt ein weiterer Wellenleitermechanismus in der Gestalt des in Fig. 20C dargestellten Filmwellenleiters hinzu, der durch die obere Hautschicht 2001 gebildet ist. Gemäß einer Ausführungsform Insbesondere ist der Netzwerkanalysator 106, 206 dazu eingerichtet, zur Bestimmung der jeweiligen Verlustgröße die komplexe Dielektrizitätszahl ε" zu ermitteln.

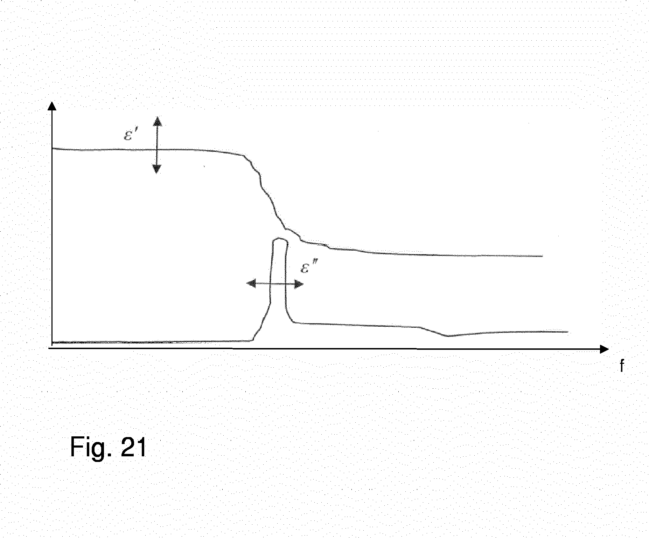

Hierzu zeigt die Fig. 21 ein Diagramm zur Illustrierung der reellen Dielektrizitätszahl ε' und der komplexen Dielektrizitätszahl ε" in Abhängigkeit der Frequenz f.

Dabei illustriert die Fig. 21, dass in dem Frequenzbereich, wo der Realteil ε' von dem höheren Niveau auf das niedrigere Niveau übergeht, die durch die komplexe Dielektrizitätszahl ε" repräsentierten Verluste steigen. Dieser Anstieg der Verluste wird in der Spektroskopie auch als Relaxationsmechanismus bezeichnet. Der Effekt, der hierbei ausgenützt werden kann, ist der, dass sich die Frequenz, bei der die Überhöhung der Verluste - siehe lokales Maximum von ε" - mit der Konzentration des Zuckergehaltes verschiebt.

Beispielsweise der menschliche Körper besteht zu 80% aus Wasser. Wasser besitzt einen Relaxationsmechanismus z.B. bei ca. 19 GHz. Deren Verstimmung kann bestimmt werden und auf den Zuckergehalt abgebildet werden. Die Verstimmung der Resonanzfrequenz bei ε" ist - wie Fig. 21 illustriert - leichter detektierbar als die Plateauveränderung von ε'. Insbesondere verschieben Variationen in der Ankopplung die Frequenz des Maximums von ε" vorteilhafterweise nicht. Damit ist eine Bestimmung der Zuckerkonzentration aus der Beobachtung von ε" deutlich weniger fehleranfällig als die Beobachtung von ε' bzw. dessen Niveauänderungen.

Weil bei einer Vielzahl von Stoffen solche Kurven wie die der Fig. 21 überlagert sind, ist eine Separation der Stoffe durch eine Beobachtung der imaginären Dielektrizitätszahl ε" einfacher durchführbar, da jedem Stoff ein bestimmtes Absorptionsmaximum zugeordnet werden kann. Bei der reellen Dielektriziätszahl ε' kann jedoch nur die Summe aller reellen Dielektrizitätszahlen ε' aller beteiligten Stoffe beobachtet werden.

Der Netzwerkanalysator 106, 206 kann ferner ausgebildet sein, eine Relaxationskonstante τ des Blutbildparameters in Abhängigkeit von der Frequenz mit der größeren oder maximalen Verlustgröße zu bestimmen. Weiter ist der Netzwerkanalysator 106, 206 dazu ausgebildet, den Blutbildparameter, wie die Glucosekonzentration im Blut, in Abhängigkeit der bestimmten Relaxationszeitkonstanten zu ermitteln.

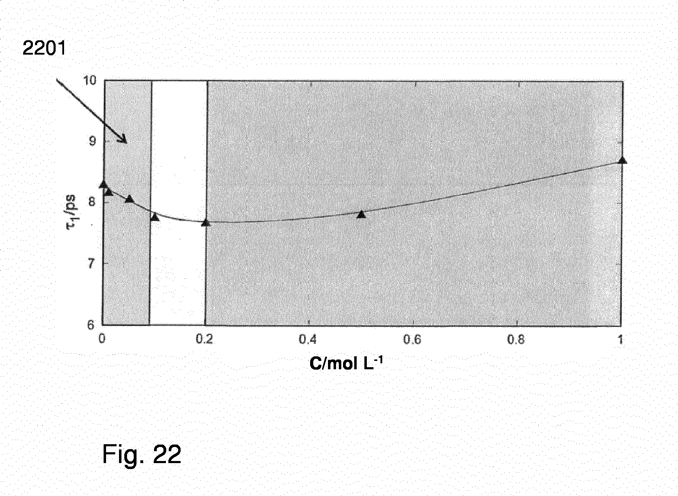

Dazu zeigt die Fig. 22 ein Diagramm zur Illustrierung eines Zusammenhangs zwischen der Relaxationszeitkonstanten (τ) und der Glucosekonzentration C/mol L$^{-1}$ im Blut. Dabei zeigt der mit dem Bezugszeichen 2201 referenzierte Bereich in der Fig. 22 einen kritischen Blutzuckerbereich.

Gemäß einer Ausführungsform ist der Netzwerkanalysator 106, 206 insbesondere dazu ausgebildet, die Relaxationszeitkonstante (τ) auf der Basis der Formel $\tau = \dfrac{1}{2\pi f_A}$ zu berechnen, wobei $f_A$ die Frequenz bezeichnet, bei welcher die ermittelte Verlustgröße maximal ist.

Vorteilhafterweise ist dann der Netzwerkanalysator 106, 206 dazu ausgebildet, die Frequenz zu bestimmen, bei welcher der Imaginärteil der komplexen Dielektrizitätszahl ε" maximal ist, und die Relaxationszeitkonstante (τ) in Abhängigkeit der bestimmten Frequenz zu ermitteln ist. Diese bestimmte Frequenz nutzt der Netzwerkanalysator 106, 206 dann zur Bestimmung des Blutbildparameters, wie der Glucosekonzentration.

**Patentansprüche**

1. Erfassungsvorrichtung (100) zum Erfassen eines Blutbildparameters von Blut in einem Blutgefäß (102), mit:

    einer elektrischen Leitungsanordnung (104), welche an das Blutgefäß (102) dielektrisch koppelbar ist;
    einem Netzwerkanalysator (106) zum Anregen der elektrischen Leitungsanordnung (104), so dass ein elektromagnetisches Feld (108) von der elektrischen Leitungsanordnung (104) ausgeht, und
    zum Erfassen einer Änderung des elektro-magnetischen Feldes (108) bei dielektrischer Belastung der elektrischen Leitungsanordnung (104) mit dem Blutgefäß (102) und zum Erfassen des Blutbildparameters auf der Basis der erfassten Änderung des elektromagnetischen Feldes (108).

2. Erfassungsvorrichtung (100) nach Anspruch 1, wobei bei dielektrischer Kopplung der elektrischen Leitungsanordnung (104) an das Blutgefäß (102) ein überwiegender Energiefluss des elektro-magnetischen Feldes (108) in dem Blutgefäß (102) konzentriert ist.

3. Erfassungsvorrichtung (100) nach Anspruch 1 oder Anspruch 2, wobei die dielektrische Kopplung der elektrischen Leitungsanordnung (104) an das Blutgefäß (102) durch nichtinvasives Befestigen der elektrischen Leitungsanordnung (104) an einer Hautoberfläche eines menschlichen oder tierischen Körpers erfolgt.

4. Erfassungsvorrichtung (100) nach einem der vorhergehenden Ansprüche, bei der der Blutbildparameter eine dielektrische ($\varepsilon_r$) und/oder magnetische ($\mu_r$) Leitfähigkeit des Blutes in dem Blutgefäß (102) umfasst.

5. Erfassungsvorrichtung (100) nach einem der vorhergehenden Ansprüche, bei der der Blutbildparameter eine Zuckerkonzentration des Blutes in dem Blutgefäß (102) umfasst.

6. Erfassungsvorrichtung (100) nach einem der vorhergehenden Ansprüche, bei der der Netzwerkanalysator (106) ausgelegt ist, den Blutbildparameter basierend auf einer S-Parametermessung zu bestimmen.

7. Erfassungsvorrichtung (100) nach einem der vorhergehenden Ansprüche, bei der der Netzwerkanalysator (106) ausgelegt ist, den Blutbildparameter basierend auf einer Messung eines Vorwärts-Transmissionsfaktors ($S_{21}$) und/ oder eines Eingangs-Reflexionsfaktors ($S_{11}$) zu bestimmen.

8. Erfassungsvorrichtung (100) nach einem der vorhergehenden Ansprüche, bei der die Änderung des elektro-magnetischen Feldes (108) eine Änderung der Ausbreitungscharakteristik des elektro-magnetischen Feldes (108) gegenüber einer Referenzcharakteristik beschreibt.

9. Erfassungsvorrichtung (100) nach Anspruch 9, bei der die Referenzcharakteristik eine Ausbreitungscharakteristik des elektro-magnetischen Feldes (108) in Wasser beschreibt.

10. Erfassungsvorrichtung (100) nach einem der vorhergehenden Ansprüche, bei der Netzwerkanalysator ausgelegt ist, den Blutbildparameter in einem Frequenzbereich von 1 bis 100 GHz zu bestimmen.

11. Erfassungsvorrichtung (100) nach einem der vorhergehenden Ansprüche, bei der die elektrischen Leitungsanordnung (104) eine Länge im Bereich von 1mm bis 20mm aufweist.

12. Erfassungsvorrichtung (100) nach einem der vorhergehenden Ansprüche, bei der die elektrischen Leitungsanordnung (104) einen halboffenen Wellenleiter, insbesondere einen geschlitzten Hohlleiter oder eine Mikrostreifenleitung umfasst.

13. Erfassungsvorrichtung (100) nach Anspruch 12, bei der der geschlitzte Hohlleiter einen Rechteckhohlleiter (1501) mit einer Mehrzahl kreisförmiger Schlitze (1503) oder mit einem rechteckförmigen Schlitz (1504), insbesondere mit einem rechteckförmigen Schlitz der Breite 1 mm und der Länge 20 mm, umfasst.

14. Erfassungsvorrichtung (100) nach Anspruch 12, bei der die Mikrostreifenleitung eine koplanare Mikrostreifenleitung (1000), insbesondere eine masselose koplanare Mikrostreifenleitung (1200) mit einer Spaltbreite im Bereich von 0,1 mm bis 0,9 mm umfasst.

15. Verfahren (1600) zum Erfassen eines Blutbildparameters von Blut in einem Blutgefäß, mit:

 dielektrisches Koppeln (1601) einer elektrischen Leitungsanordnung an das Blutgefäß;
 Anregen (1602) der elektrischen Leitungsanordnung, so dass ein elektro-magnetisches Feld von der elektrischen Leitungsanordnung ausgeht;
 Erfassen (1603) einer Änderung des elektro-magnetischen Feldes bei dielektrischer Belastung der elektrischen Leitungsanordnung mit dem Blutgefäß; und
 Erfassen (1604) des Blutbildparameters auf der Basis der erfassten Änderung des elektro-magnetischen Feldes.

Fig. 1

Fig. 2

einfallende Welle, a1

T1

reflektierte Welle, b1

T2

$\varepsilon_{r1} , \mu_{r1}$          $\varepsilon_{r2} , \mu_{r2}$

Blutgefäß

102

300a

einfallende Welle, a1                    übertragene Welle, b2

T1

reflektierte Welle, b1

T2

$\varepsilon_{r1} , \mu_{r1}$     $\varepsilon_{r2} , \mu_{r2}$     $\varepsilon_{r1} , \mu_{r1}$

Blutgefäß

102

300b

Fig. 3

Fig. 4

Fig. 5

Fig. 6

Fig. 7

Fig. 8

900

903

901

902

903

$\Delta arg(s_{21})/mm$

902

901

Frequenz in [GHz]

Fig. 9

Draufsicht

1003

1001

1000

Seitenansicht

1003

1001

Fig. 10

Fig. 11

Fig. 12

1300

1301

Fig. 13

1400

$\Delta\arg(s_{21})$

Fig. 14

1500

1501     1502

1503

1504

Fig. 15

1600

| Dielektrisches Koppeln | 1601 |

| Anregen | 1602 |

| Erfassen | 1603 |

| Erfassen | 1604 |

Fig. 16

Fig. 17

Fig. 18

| Durchm. El | A | B | C | D | E | F | G |
|---|---|---|---|---|---|---|---|
| 6 mm | <1 | +2 | -1 | -9 | -17 | +11 | <1 |
| 3,4 mm | <1 | +4 | -1 | -13 | - | - | -10 |

Fig. 19

2001

Haut

2003 Vene

=

Fettgewebe 2005

**Fig. 20A**

2001

2003

Vene

+

Fettgewebe 2005

**Fig. 20B**

2001

Haut

2005

Fettgewebe

**Fig. 20C**

Fig. 21

Fig. 22

Europäisches
Patentamt

European
Patent Office

Office européen
des brevets

## EUROPÄISCHER RECHERCHENBERICHT

Nummer der Anmeldung

EP 11 19 0552

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2010/131029 A1 (MICROSENSE) 18. November 2010 (2010-11-18) * Seiten 7-8 * * Seiten 10-12 * ----- | 1-15 | INV. A61B5/145 A61B5/05 |
| | | | RECHERCHIERTE SACHGEBIETE (IPC) |
| | | | A61B |

Der vorliegende Recherchenbericht wurde für alle Patentansprüche erstellt

| Recherchenort | Abschlußdatum der Recherche | Prüfer |
|---|---|---|
| München | 13. Februar 2012 | Martelli, Luca |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 11 19 0552

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-02-2012

| Im Recherchenbericht angeführtes Patentdokument | Datum der Veröffentlichung | Mitglied(er) der Patentfamilie | Datum der Veröffentlichung |
|---|---|---|---|
| WO 2010131029 A1 | 18-11-2010 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 10192470 A
- EP 10192473 A
- EP 10192472 A


**In der Beschreibung aufgeführte Nicht-Patentliteratur**

- **BUFORD RANDAL JEAN et al.** A microwave frequency sensor for non-invasive blood-glucose measurement. *SAS 2008 - IEEE Sensors Applications Symposium,* 12. Februar 2008
- Calibration methodology for a microwave non-invasive glucose sensor. **M. MCCLUNG.** Master Thesis. Baylor University, Mai 2008
- **NETTER, F. N.** Atlas der Anatomie. Thieme Verlag, 2006